# EUROPÄISCHE PATENTSCHRIFT

(11) **EP 3 897 693 B1**
(45) Veröffentlichungstag und Bekanntmachung des Hinweises auf die Patenterteilung: **06.08.2025**
(21) Anmeldenummer: 19835657.8
(22) Anmeldetag: 20.12.2019
(51) Int. Cl.: A61K 38/39, A23L 33/18, A61K 38/04, A61P 19/02, A61P 19/08, A61P 19/10, A61P 1/00, A61P 9/00, A61P 21/00, A61P 25/28, A61P 17/00, C07K 14/78, A61K 8/65, A23L 29/281, A61Q 19/08

(54) **SYNTHETISCHE UND REKOMBINANT HERGESTELLTE KOLLAGENPEPTIDE MIT BIOLOGISCHER WIRKSAMKEIT**
SYNTHETIC AND RECOMBINANT COLLAGEN PEPTIDES HAVING BIOLOGICAL ACTIVITY
PEPTIDES DE COLLAGÈNE DE SYNTHÈSE ET OBTENUS DE MANIÈRE RECOMBINÉE À EFFICACITÉ BIOLOGIQUE

(30) Priorität: 21.12.2018 DE 102018222898; 26.02.2019 DE 102019202608; 28.05.2019 DE 102019207859
(43) Veröffentlichungstag der Anmeldung: 27.10.2021
(73) Patentinhaber: GELITA AG, 69412 Eberbach (DE)
(72) Erfinder: HAUSMANNS, Stephan, 69126 Heidelberg (DE); FRECH, Hans-Ulrich, 69469 Weinheim (DE); OESSER, Steffen, 24960 Glücksburg (DE); HAHN, Martin, 48599 Gronau (DE)
(74) Vertreter: Schrell, Andreas
(86) Internationale Anmeldenummer: PCT/EP2019/086613
(87) Internationale Veröffentlichungsnummer: WO 2020/127929

(56) Entgegenhaltungen:
- WO-A1-2018/041684
- WO-A2-01/34801
- WO-A2-2006/052451
- WO-A2-2007/087131
- DE-A1- 102009 030 351
- DE-A1- 102011 000 997
- DE-A1- 102018 120 183
- OLSEN D ET AL: "Expression and characterization of a low molecular weight recombinant human gelatin: development of a substitute for animal-derived gelatin with superior features", PROTEIN EXPRESSION AND PURIFICATION, ACADEMIC PRESS, SAN DIEGO, CA, vol. 40, no. 2, 1 April 2005 (2005-04-01), pages 346 - 357, XP004781387, ISSN: 1046-5928, DOI: 10.1016/J.PEP.2004.11.016

## Beschreibung

Die vorliegende Erfindung betrifft ein synthetisch oder rekombinant hergestelltes Kollagenpeptid zur Anwendung in einem Verfahren zur therapeutischen Behandlung des menschlichen oder tierischen Körpers mit einem Molekulargewicht in einem Bereich von 0,18 bis 5,0 kDa, insbesondere 1,1 bis 5,0 kDa sowie Produkte, die ein erfindungsgemäßes Kollagenpeptid umfassen.

Kollagen ist ein extrazelluläres Strukturprotein, das in Tieren vorkommt, zum Beispiel in Säugetieren, Vögeln und Fischen. Es findet sich dort in der Regel im Bindegewebe, insbesondere als Bestandteil der extrazellulären Matrix. Besonders reich an Kollagen sind Sehnen, Bänder, Knorpel und Knochen. In Pflanzen und einzelligen Organismen sind Kollagene hingegen nicht anzutreffen.

Kollagene treten in verschiedenen, strukturell und funktionell unterschiedlichen Typen auf und unterscheiden sich unter anderem in Hinblick auf ihre Struktur, Funktion und Herkunft. Die das Kollagen aufbauenden Polypeptidketten werden in der Zelle einzeln an den Ribosomen des endoplasmatischen Retikulums in Form größerer Vorläufermoleküle synthetisiert und weisen umfangreiche repetitive (Gly-X-Y)ₙ-Sequenzen auf, wobei X und Y jede Aminosäure sein können, meist allerdings Prolin und 4-Hydroxyprolin sind.

Diese Vorläufer-Polypeptidketten werden im endoplasmatischen Retikulum unter Bildung von Hydroxyprolin- und Hydroxylysinresten posttranslational an Prolin- und Lysinresten der Polypeptidkette hydroxyliert. Die Hydroxylierung dient der Stabilisierung benachbarter Kollagen-Polypeptidketten der sich in der Zelle bildenden rechtsgängigen Tripel-Helix aus jeweils drei der Vorläufer-Polypeptidketten (Prokollagen).

Das so gebildete Prokollagen wird intrazellulär glykosyliert, in der glykosylierten tripelhelikalen Form (Tropokollagen) von der Zelle sezerniert und anschließend durch Peptidase-vermittelte Abspaltung der terminalen Reste Kollagen gebildet. Dieses lagert sich im Rahmen eines Fibrillogenese-Prozesses zu Kollagenfibrillen zusammen, die anschließend kovalent quervernetzt werden und Kollagenfasern bilden.

Kollagen wird häufig auch in denaturierter und dann als Gelatine bezeichneter Form oder in Form von Hydrolysaten genutzt.

Unterzieht man Gelatine oder Kollagen zum Erhalt von Kollagenpeptiden hydrolytischen Prozessen, insbesondere einer enzymatischen Hydrolyse, können je nach eingesetztem Kollagentyp und Herkunft sowie enzymatischen Bedingungen Kollagenhydrolysate mit unterschiedlichster Zusammensetzung und Anwendungsprofil hergestellt werden. Diese Kollagenhydrolysate stellen jedoch ein Gemisch von Peptiden dar, deren Molekulargewichte über bestimmte Größenbereiche verteilt sind. Die Verwendung solcher Kollagenhydrolysate zum Beispiel als Nahrungsergänzungsmittel oder als kosmetisches Hilfsmittel ist seit längerem bekannt, unter anderem zur Vorbeugung und/oder Behandlung von Beschwerden, die im Zusammenhang mit den Knochen, den Gelenken oder dem Bindegewebe stehen.

So beschreibt die WO 2012/065782 aus Schweinschwartengelatine gewonnene Kollagenhydrolysate, die zur Stimulation der Biosynthese extrazellulärer Matrixproteine durch Hautzellen dienen und insbesondere für kosmetische Zwecke geeignet sind.

Die WO 2012/117012 offenbart enzymatisch hydrolysiertes Kollagen aus Rinderspalt mit einem mittleren Molekulargewicht von 1500 bis 8000 Da, das gemeinsam mit einem Präbiotikum zur Vorbeugung und/oder Behandlung von Osteoporose eingesetzt werden kann.

Die WO 2018/041684 offenbart die Verwendung von Kollagenhydrolysat zur Verbesserung der Ausdauerleistungsfähigkeit und Stimulation des Fettabbaus.

Obgleich für viele Anwendungen und Konsumentengruppen die Verwendung von aus tierischen Materialien gewonnen Kollagenhydrolysaten Vorteile mit sich bringt, kann mit Blick auf bestimmte Konsumentengruppen und Anwendungsprofile die Verwendung solchermaßen gewonnener Kollagenhydrolysate auch weniger wünschenswert sein. So stehen bestimmte Konsumentengruppen aus tierischen Materialien gewonnenen Rohstoffen grundsätzlich kritisch oder ablehnend gegenüber, sei es, dass Kontaminationen mit gesundheitsgefährdenden Mikroorganismen oder Agentien, zum Beispiel Verfahrenshilfsstoffen, oder unerwünschte Immunreaktionen befürchtet werden oder aus religiösen oder ethischen Motiven. Darüber hinaus umfassen die für die Gewinnung von aus tierischen Materialien gewonnenen Kollagenhydrolysaten eingesetzten Herstellverfahren häufig aufwändige und teure Aufschluss-, Reinigungs- und Weiterverarbeitungsschritte.

Vor diesem Hintergrund ist es nicht verwunderlich, dass Verfahren entwickelt wurden, Gelatine, Kollagen, Kollagenhydrolysate und einzelne Kollagenpeptide auf biotechnologischem Wege unter Einsatz rekombinanter Gentechnologie herzustellen.

So offenbart die WO 2006/052451 A2 die Herstellung von rekombinantem Kollagen Typ III in *Pichia pastoris*-Stämmen, die auch humane Prolylhydroxylasen exprimieren.

Die WO 2005/012356 A2 offenbart die Herstellung von Gelatine aus humanem Kollagen Typ I sowie einzelner 50 kDa, 65 kDa und 100 kDa großer Kollagenpeptidspezies, jeweils in vollständig hydroxylierter, partiell und nicht-hydroxylierter Form.

Olsen et al. (Protein Expression and Purification, 2005, 40, pg. 346-357) offenbart die rekombinante Herstellung einer 8,5 kDa großen Kollagenpeptidspezies aus der α1-Kette humanen Kollagens in *Pichia pastoris.*

Die WO 01/34646 A2 offenbart ebenfalls die Herstellung einzelner rekombinanter Gelatinespezies mit sich jeweils aus dem rekombinanten Herstellweg ergebendem definiertem Molekulargewicht von 0 bis 350 kDa, die in nicht-hydroxylierter, partiell oder vollständig hydroxylierter Form vorliegen können.

Die Druckschrift offenbart die allgemeine Verwendbarkeit einzelner rekombinanter Gelatinespezies oder von deren Gemischen in der Nahrungsmittel-, Getränke-, Kosmetik- oder Pharmaindustrie. Die Druckschrift offenbart weitere Verwendungsmöglichkeiten der offenbarten rekombinanten Produkte als photographische Zusammensetzung oder als technisches Hilfsmittel, z.B. in der Halbleiterindustrie, Papierherstellung oder ähnlichem.

Bekannt ist es daher, rekombinante Gelatine- oder Kollagenpeptide beliebiger Größe einzeln oder im Gemisch hydroxyliert oder nicht-hydroxyliert in verschiedensten technischen oder nichttechnischen Bereichen, beispielsweise in der Nahrungsmittelindustrie oder in der Therapie menschlicher oder tierischer Erkrankungen einzusetzen.

Auch mit Blick auf die in weiten Teilen der Bevölkerung gestiegenen Ansprüche in Hinblick auf Gesundheit, attraktives äußeres Erscheinungsbild, Mobilität und Fitness, jeweils auch im fortgeschrittenen Alter, besteht jedoch nach wie vor ein großer Bedarf an Nahrungsmitteln und Präparaten für die Verbesserung und/oder den Erhalt der Gesundheit sowie für die Therapie von Krankheiten.

Gesundheit, Mobilität, körperliche Attraktivität und körperliche Fitness hängen häufig eng zusammen mit dem Zustand des Bindegewebes. Bindegewebe sind Gewebe, die in allen Geweben des Körpers, beispielsweise in den Muskeln, dem Darm, den Blutgefäßen, dem Zahnhalteapparat, Sehnen, Bändern, Knochen und der Haut, vorkommen und dort häufig unterstützende Aufgaben verrichten. Bindegewebe weisen in der Regel vergleichsweise wenig Zellen und viel Zellzwischensubstanz auf, die in der Regel extrazelluläre Matrixproteine, insbesondere Kollagen enthält. Bindegewebe können insbesondere in Form von lockerem Bindegewebe, straffem Bindegewebe, zum Beispiel in der Haut, Sehnen, Bändern und der Gehirnhaut, gallertigem Bindegewebe und verschiedenen anderen Bindegewebstypen, insbesondere auch Stützgeweben, nämlich Knorpelgewebe und Knochengewebe, auftreten und sie sind insbesondere auch für die Mobilität, das äußere Erscheinungsbild, die Integrität der körperinneren Strukturen und die Fitness eines Körpers entscheidend.

Das der vorliegenden Erfindung zugrundeliegende technische Problem liegt daher darin, Mittel und Verfahren bereitzustellen, die eine biologische Wirksamkeit in Hinblick auf Bindegewebe, insbesondere Bindegewebe der Haut, Sehnen, Bänder, Gehirnhaut, Knorpel und Knochen, aufweisen und es so erlauben, einerseits mit diesem Bindegewebe zusammenhängende Erkrankungen zu behandeln und andererseits einen als erwünscht angesehenen Zustand derartiger Bindegewebe zu erhalten.

Der vorliegenden Erfindung liegt auch das technische Problem zugrunde, synthetische oder rekombinante Kollagenpeptide bereitzustellen, die die vorgenannten Nachteile überwinden, insbesondere die sich in standardisierter, verlässlicher und genau definierter Form rekombinant, auch im größerem industriellen und kostengünstigen Maßstab, herstellen lassen und sich aufgrund ihrer biologischen Wirksamkeit zur Anwendung in einem Verfahren zur therapeutischen Behandlung des menschlichen oder tierischen Körpers eignen.

Die vorliegende Erfindung löst das ihr zugrunde liegende technische Problem durch die Bereitstellung der Lehren der unabhängigen Patentansprüche, insbesondere auch der Lehren der bevorzugten Ausführungsformen in der Beschreibung und der abhängigen Patentansprüche.

Die vorliegende Erfindung betrifft ein synthetisches oder rekombinantes Kollagenpeptid zur Anwendung in einem Verfahren zur Prävention und/oder Behandlung von Knochenerkrankungen, Knorpelerkrankungen, degenerativen Gelenkerkrankungen, Erkrankungen der Sehnen oder Bänder und/oder Behandlung von Hauterkrankungen, wobei das Kollagenpeptid ein Molekulargewicht in einem Bereich von 0,18 bis 5,0 kDa aufweist und in der Lage ist, in Bindegewebszellen, insbesondere in Osteoblasten, Chondrocyten und/oder Fibroblasten, die Synthese von extrazellulären Matrixproteinen, insbesondere Kollagen, zu stimulieren und wobei das Kollagenpeptid eine Aminosäuresequenz ausgewählt aus der Gruppe bestehend aus SEQ ID No. 1 bis 30 aufweist.

Die erfindungsgemäß beanspruchten synthetischen oder rekombinanten Kollagenpeptide, insbesondere synthetisch oder rekombinant hergestellten Kollagenpeptide, mit einem Molekulargewicht in einem Bereich von 0,18 bis 5,0 kDa, insbesondere 1,1 bis 5,0 kDa, zeigen vorteilhafterweise eine biologische Wirksamkeit, insbesondere in Bindegewebe der Sehnen und Bänder, insbesondere Knorpelgewebe und Knochengewebe, und eignen sich dadurch insbesondere zur Anwendung in einem Verfahren zur therapeutischen oder nicht-therapeutischen Behandlung des menschlichen oder tierischen Körpers. Gemäß einer bevorzugten Ausführungsform der vorliegenden Erfindung weist das synthetische oder rekombinante Kollagenpeptid, insbesondere das synthetisch oder rekombinant hergestellte Kollagenpeptid, die Aminosäuresequenz SEQ ID No. 1, SEQ ID No. 6 auf, bevorzugt besteht das erfindungsgemäße Kollagenpeptid aus einer dieser Aminosäuresequenzen.

Bevorzugt weist das synthetische oder rekombinante Kollagenpeptid, insbesondere das synthetisch oder rekombinant hergestellte Kollagenpeptid, die Aminosäuresequenz SEQ ID No. 1 auf, bevorzugt besteht das erfindungsgemäße Kollagenpeptid aus dieser Aminosäuresequenz.

Bevorzugt weist das synthetische oder rekombinante Kollagenpeptid, insbesondere das synthetisch oder rekombinant hergestellte Kollagenpeptid, die Aminosäuresequenz SEQ ID No. 6 auf, bevorzugt besteht das erfindungsgemäße Kollagenpeptid aus dieser Aminosäuresequenz.

In einer bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt aus SEQ ID No. 1 bis 5, insbesondere ausgewählt aus SEQ ID No. 1 bis 4, auf, bevorzugt besteht das erfindungsgemäße Kollagenpeptid aus einer dieser Aminosäuresequenzen. Gemäß dieser Ausführungsform weist das synthetische oder rekombinante Kollagenpeptid, insbesondere das synthetisch oder rekombinant hergestellte Kollagenpeptid, demnach kein Hydroxyprolin in seiner Aminosäuresequenz auf.

In einer bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt aus SEQ ID No. 1 bis 5, insbesondere ausgewählt aus SEQ ID No. 1 bis 4, auf, bevorzugt besteht das erfindungsgemäße Kollagenpeptid aus einer dieser Aminosäuresequenzen, wobei mindestens ein Prolyl- und/oder mindestens ein Lysylrest hydroxyliert ist. Gemäß dieser Ausführungsform weist das synthetische oder rekombinante Kollagenpeptid, insbesondere das synthetisch oder rekombinant hergestellte Kollagenpeptid, demnach mindestens ein Hydroxyprolin und/oder mindestens ein Hydroxylysin in seiner Aminosäuresequenz auf.

In einer weiteren bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt aus SEQ ID No. 6 bis 10, insbesondere ausgewählt aus SEQ ID No. 6 bis 9, auf, bevorzugt besteht das erfindungsgemäße Kollagenpeptid aus einer dieser Aminosäuresequenzen.

Gemäß einer weiteren bevorzugten Ausführungsform weist das synthetische oder rekombinante Kollagenpeptid, insbesondere das synthetisch oder rekombinant hergestellte Kollagenpeptid, eine Aminosäuresequenz ausgewählt aus der Gruppe bestehend aus SEQ ID No. 1 bis 10, insbesondere ausgewählt aus der Gruppe bestehend aus SEQ ID No. 1 bis 4 und 6 bis 9 auf.

Bevorzugt besteht das synthetische oder rekombinante Kollagenpeptid, insbesondere das synthetisch oder rekombinant hergestellte Kollagenpeptid, aus einer der Aminosäuresequenz ausgewählt aus der Gruppe bestehend aus SEQ ID No. 1 bis 10, insbesondere ausgewählt aus der Gruppe bestehend aus SEQ ID No. 1 bis 4 und 6 bis 9.

Gemäß einer weiteren bevorzugten Ausführungsform der vorliegenden Erfindung weist das synthetische oder rekombinante Kollagenpeptid, insbesondere das synthetisch oder rekombinant hergestellte Kollagenpeptid, die Aminosäuresequenz SEQ ID No. 15, SEQ ID No. 21 auf, bevorzugt besteht das erfindungsgemäße Kollagenpeptid aus einer dieser Aminosäuresequenzen.

Bevorzugt weist das synthetische oder rekombinante Kollagenpeptid, insbesondere das synthetisch oder rekombinant hergestellte Kollagenpeptid, die Aminosäuresequenz SEQ ID No. 15 auf, bevorzugt besteht das erfindungsgemäße Kollagenpeptid aus dieser Aminosäuresequenz.

Bevorzugt weist das synthetische oder rekombinante Kollagenpeptid, insbesondere das synthetisch oder rekombinant hergestellte Kollagenpeptid, die Aminosäuresequenz SEQ ID No. 21 auf, bevorzugt besteht das erfindungsgemäße Kollagenpeptid aus dieser Aminosäuresequenz.

In einer bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt aus SEQ ID No. 15 und SEQ ID No. 16 auf, bevorzugt besteht das erfindungsgemäße Kollagenpeptid aus einer dieser Aminosäuresequenzen. Gemäß dieser Ausführungsform weist das synthetische oder rekombinante Kollagenpeptid, insbesondere das synthetisch oder rekombinant hergestellte Kollagenpeptid, demnach kein Hydroxyprolin in seiner Aminosäuresequenz auf.

In einer bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt SEQ ID No. 15 und SEQ ID No. 16 auf, bevorzugt besteht das erfindungsgemäße Kollagenpeptid aus einer dieser Aminosäuresequenzen, wobei mindestens ein Prolyl- und/oder mindestens ein Lysylrest hydroxyliert ist. Gemäß dieser Ausführungsform weist das synthetische oder rekombinante Kollagenpeptid, insbesondere das synthetisch oder rekombinant hergestellte Kollagenpeptid, demnach mindestens ein Hydroxyprolin und/oder mindestens ein Hydroxylysin in seiner Aminosäuresequenz auf.

In einer weiteren bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt aus SEQ ID No. 21 und 22 auf, bevorzugt besteht das erfindungsgemäße Kollagenpeptid aus einer dieser Aminosäuresequenzen.

Besonders bevorzugt weist das synthetische oder rekombinante Kollagenpeptid, insbesondere das synthetisch oder rekombinant hergestellte Kollagenpeptid, die Aminosäuresequenz SEQ ID No. 1, SEQ ID No. 15 auf, bevorzugt besteht das erfindungsgemäße Kollagenpeptid aus einer dieser Aminosäuresequenzen.

Gemäß einer weiteren bevorzugten Ausführungsform weist das synthetische oder rekombinante Kollagenpeptid, insbesondere das synthetisch oder rekombinant hergestellte Kollagenpeptid, eine Aminosäuresequenz ausgewählt aus der Gruppe bestehend aus SEQ ID No. 1 bis 4, 6 bis 9, 12 bis 14 und 18 bis 20 auf.

In einer weiteren bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz gemäß SEQ ID No. 7 auf.

Bevorzugt besteht das synthetische oder rekombinante Kollagenpeptid, insbesondere das synthetisch oder rekombinant hergestellte Kollagenpeptid, aus der Aminosäuresequenz gemäß SEQ ID No. 7.

In einer weiteren bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt aus SEQ ID No. 17 bis 19, 21 und 22 auf.

Bevorzugt besteht das synthetische oder rekombinante Kollagenpeptid, insbesondere das synthetisch oder rekombinant hergestellte Kollagenpeptid, aus der Aminosäuresequenz ausgewählt aus SEQ ID No. 17 bis 19, 21 und 22.

In einer weiteren bevorzugten Ausführungsform der vorliegenden Erfindung weist das synthetische oder rekombinante Kollagenpeptid, insbesondere das synthetisch oder rekombinant hergestellte Kollagenpeptid, eine Aminosäuresequenz ausgewählt aus SEQ ID No. 27 bis 30 auf.

Bevorzugt besteht das synthetische oder rekombinante Kollagenpeptid, insbesondere das synthetisch oder rekombinant hergestellte Kollagenpeptid, aus der Aminosäuresequenz ausgewählt aus SEQ ID No. 27 bis 30.

In besonders bevorzugter Ausführungsform handelt es sich bei dem synthetischen oder rekombinanten Kollagenpeptid, insbesondere dem synthetisch oder rekombinant hergestellten Kollagenpeptid, gemäß der vorliegenden Erfindung um ein nicht-hydroxyliertes Kollagenpeptid.

In einer weiteren bevorzugten Ausführungsform handelt es sich bei dem synthetischen oder rekombinanten Kollagenpeptid, insbesondere dem synthetisch oder rekombinant hergestellten Kollagenpeptid, gemäß der vorliegenden Erfindung um ein hydroxyliertes Kollagenpeptid.

Bevorzugt weist das synthetische oder rekombinante Kollagenpeptid, insbesondere das synthetisch oder rekombinant hergestellte Kollagenpeptid, hydroxyliertes Prolin und/oder hydroxyliertes Lysin, auf.

Bevorzugt ist das synthetische oder rekombinante Kollagenpeptid, insbesondere das synthetisch oder rekombinant hergestellte Kollagenpeptid, ein nicht-hydroxyliertes, partiell hydroxyliertes oder vollständig hydroxyliertes Kollagenpeptid. Gemäß einer weiteren besonders bevorzugten Ausführungsform ist jedes in dem synthetischen oder rekombinanten Kollagenpeptid vorliegende Prolin hydroxyliert.

Gemäß einer bevorzugten Ausführungsform der vorliegenden Erfindung ist das synthetische oder rekombinante Kollagenpeptid, insbesondere das synthetisch oder rekombinant hergestellte Kollagenpeptid, glykosyliert. Bevorzugt ist das synthetische oder rekombinante Kollagenpeptid, insbesondere das synthetisch oder rekombinant hergestellte Kollagenpeptid, an mindestens einem hydroxylierten Lysin glykosyliert. Bevorzugt ist jedes hydroxylierte Lysin des synthetischen oder rekombinanten Kollagenpeptid, insbesondere des synthetisch oder rekombinant hergestellten Kollagenpeptids, glykosyliert.

In einer bevorzugten Ausführungsform der vorliegenden Erfindung weist das erfindungsgemäße Kollagenpeptid eine in Kollagen der Typen I, II, III, IV, V, VI, VII, VIII, IX, X, XI, XII, XIII, XIV, XV, XVI, XVII, XVIII, XIX, XX, XXI, XXII, XXIII, XXIV, XXV, XXVI, XXVII, bevorzugt Typ I, II oder III, bevorzugt Typ I, bevorzugt Typ II, bevorzugt Typ III, vorkommende Aminosäuresequenz auf. Bevorzugt weist das erfindungsgemäße Kollagenpeptid eine in Kollagen aus Wirbeltieren, insbesondere aus Fischen, Amphibien, Reptilien, Vögeln und Säugetieren, insbesondere in humanem, bovinem, porcinem, equinem oder avianem Kollagen der Typen I, II oder III, bevorzugt Typ I, bevorzugt Typ II, bevorzugt Typ III, vorkommende Aminosäuresequenz auf.

Besonders bevorzugt weist das erfindungsgemäße Kollagenpeptid eine in humanem Kollagen, insbesondere in humanen Typ I Kollagen, bevorzugt in der α1-Kette des humanen Typ I Kollagen, vorkommende Aminosäuresequenz auf.

Besonders bevorzugt weist das erfindungsgemäße Kollagenpeptid eine in nicht-humanem Kollagen, insbesondere in nicht-humanem Typ I Kollagen, bevorzugt in der α1-Kette des nichthumanen Typ I Kollagens vorkommende Aminosäuresequenz auf, insbesondere eine in bovinem, porcinem, equinem oder avianem Kollagen vorkommende Aminosäuresequenz.

In einer bevorzugten Ausführungsform der vorliegenden ist das synthetische oder rekombinante Kollagenpeptid, insbesondere das synthetisch oder rekombinant hergestellte Kollagenpeptid, Kollagenase-resistent, insbesondere resistent gegen die Verdauung durch humane Kollagenasen.

Gemäß einer weiteren bevorzugten Ausführungsform der vorliegenden Erfindung ist das synthetische oder rekombinante Kollagenpeptid, insbesondere das synthetisch oder rekombinant hergestellte Kollagenpeptid, dazu in der Lage, die Synthese von extrazellulären Matrixproteinen, wie Kollagen, Proteoglykan und/oder Elastin, insbesondere von Kollagen, in Bindegewebszellen, insbesondere in Osteoblasten, Chondrocyten und/oder Fibroblasten, zu stimulieren.

In einer bevorzugten Ausführungsform der vorliegenden Erfindung ist das erfindungsgemäße Kollagenpeptid ein synthetisch hergestelltes Kollagenpeptid, bevorzugt ein durch chemische Synthese, insbesondere Festphasensynthese, bevorzugt Merrifield-Synthese, Bailey-PeptidSynthese oder chemoenzymatische Synthese hergestelltes Kollagenpeptid. In einer weiteren bevorzugten Ausführungsform der vorliegenden Erfindung ist das erfindungsgemäße Kollagenpeptid ein rekombinant hergestelltes Kollagenpeptid.

In einer bevorzugten Ausführungsform der vorliegenden Erfindung weist das synthetische oder rekombinante Kollagenpeptid, insbesondere das synthetisch oder rekombinant hergestellte Kollagenpeptid, keine Aminosäure-Modifikation auf, insbesondere keine Hydroxylierung auf. Besonders bevorzugt weist das synthetische oder rekombinante Kollagenpeptid, insbesondere das synthetisch oder rekombinant hergestellte Kollagenpeptid, keine hydroxylierten und/oder glykosylierten Aminosäuren auf.

Gemäß einer bevorzugten Ausführungsform der vorliegenden Erfindung weist das synthetische oder rekombinante Kollagenpeptid, insbesondere das synthetisch oder rekombinant hergestellte Kollagenpeptid, zur Anwendung in einem Verfahren zur therapeutischen Behandlung des menschlichen oder tierischen Körpers ein Molekulargewicht in einem Bereich von 0,18 bis 5,0 kDa, bevorzugt 0,2 bis 5,0 kDa, bevorzugt 0,3 bis 5,0 kDa, bevorzugt 0,4 bis 5,0 kDa, bevorzugt 0,5 bis 5,0 kDa, bevorzugt 0,6 bis 5,0 kDa, bevorzugt 0,7 bis 5,0 kDa, bevorzugt 0,8 bis 5,0 kDa, bevorzugt 0,9 bis 5,0 kDa, bevorzugt 1,0 bis 5,0 kDa, bevorzugt 1,1 bis 5,0 kDa, bevorzugt 1,2 bis 5,0 kDa, bevorzugt 1,3 bis 5,0 kDa, bevorzugt 1,4 bis 5,0 kDa, bevorzugt 1,5 bis 5,0 kDa, bevorzugt 1,6 bis 5,0 kDa, bevorzugt 1,7 bis 5,0 kDa, bevorzugt 1,8 bis 5,0 kDa, bevorzugt 1,9 bis 5,0 kDa, bevorzugt 2,0 bis 5,0 kDa, bevorzugt 2,1 bis 4,9 kDa, bevorzugt 2,2 bis 4,8 kDa, bevorzugt 2,3 bis 4,7 kDa, bevorzugt 2,4 bis 4,6 kDa, bevorzugt 2,5 bis 4,5 kDa, bevorzugt 1,2 bis 3,2, bevorzugt 1,3 bis 3,0 kDa, bevorzugt 1,5 bis 3,0 kDa, bevorzugt 1,8 bis 3,0 kDa, bevorzugt 2,0 bis 3,0 kDa, auf.

Die vorliegende Erfindung betrifft zudem das erfindungsgemäße synthetische oder rekombinante Kollagenpeptid, insbesondere das synthetisch oder rekombinant hergestellte Kollagenpeptid, zur Anwendung in einem Verfahren zur Prävention und/oder Behandlung von Knochenerkrankungen, insbesondere Osteoporose.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung von Sarkopenie.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung von degenerativem Verlust von Muskelmasse.

In bevorzugter Ausführungsform betrifft die vorliegende Erfindung das erfindungsgemäße synthetische oder rekombinante Kollagenpeptid, insbesondere das synthetisch oder rekombinant hergestellte Kollagenpeptid, zur Anwendung in einem Verfahren zur Prävention und/oder Behandlung von Knorpelerkrankungen, insbesondere Arthrose oder Arthritis.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Verbesserung der Muskelkraft.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung eines pathologischen Zustandes charakterisiert durch eine verminderte mitochondriale Aktivität, insbesondere zur Prävention und/oder Behandlung eines pathologischen Zustandes charakterisiert durch eine verminderte Ausdauerleistungsfähigkeit.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Stimulation des Fettabbaus.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Reduktion des Körpergewichtes.

In bevorzugter Ausführungsform betrifft die vorliegende Erfindung das erfindungsgemäße synthetische oder rekombinante Kollagenpeptid, insbesondere das synthetisch oder rekombinant hergestellte Kollagenpeptid, zur Anwendung in einem Verfahren zur Prävention und/oder Behandlung von Arthrose, rheumatoider Arthritis, rheumatischen Erkrankungen, Spondylitis und/oder Fibromyalgie.

In bevorzugter Ausführungsform betrifft die vorliegende Erfindung das erfindungsgemäße synthetische oder rekombinante Kollagenpeptid, insbesondere das synthetisch oder rekombinant hergestellte Kollagenpeptid, zur Anwendung in einem Verfahren zur Prävention und/oder Behandlung von Psoriasis vulgaris, Akne, atopische Dermatitis, chronische Pruritus und/oder Rosazea.

In bevorzugter Ausführungsform betrifft die vorliegende Erfindung das erfindungsgemäße synthetische oder rekombinante Kollagenpeptid, insbesondere das synthetisch oder rekombinant hergestellte Kollagenpeptid, zur Anwendung in einem Verfahren zur Behandlung von Wunden, insbesondere von chronischen Wunden, akuten Wunden und/oder Brandwunden.

Offenbart (nicht erfindungsgemäß) werden synthetische oder rekombinante Kollagenpeptide, insbesondere das synthetisch oder rekombinant hergestellte Kollagenpeptid, zur Anwendung in einem Verfahren zur Prävention und/oder Behandlung von degenerativen Nervenerkrankungen.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung von Demenz.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung der Alzheimer-Erkrankung.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung eines pathologischen Zustandes charakterisiert durch eine Verminderung der mentalen Leistungsfähigkeit.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung von mit Fehlfunktionen der Blut-Hirn-Schranke, insbesondere der Struktur und/oder Funktion der Hirnhaut, zusammenhängenden Krankheiten.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung von Darmerkrankungen, insbesondere chronisch-entzündlichen Darmerkrankungen.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung von Erkrankungen des Herz-KreislaufSystems, insbesondere der Struktur und/oder Funktion der Blutgefäße, insbesondere der Gefäßwand, insbesondere zur Prävention und/oder Behandlung von Bluthochdruck und/oder Durchblutungsstörungen.

Offenbart (nicht erfindungsgemäß) werden synthetische oder rekombinante Kollagenpeptid, insbesondere das synthetisch oder rekombinant hergestellte Kollagenpeptid, zur Anwendung in einem Verfahren zur Prävention und/oder Behandlung von Erkrankungen des Zahnhalteapparats.

Die vorliegende Erfindung betrifft ferner ein nicht-therapeutisches Verfahren unter Verwendung des erfindungsgemäßen Kollagenpeptids zur optischen Verbesserung der Haut, insbesondere zur Reduktion der Faltenbildung, Verbesserung der Hautelastizität, Erhöhung der Spannkraft der Haut, Erhöhung des Feuchtigkeitsgehalts der Haut, Reduktion von Cellulite und/oder Reduktion von Dehnungssteifen, insbesondere Schwangerschaftsstreifen.

Die vorliegende Erfindung betrifft auch ein nicht-therapeutisches Verfahren unter Verwendung des erfindungsgemäßen Kollagenpeptids zur Beschleunigung des Wachstums der Nägel und/oder Reduktion der Brüchigkeit von Nägeln.

Die vorliegende Erfindung betrifft zudem ein nicht-therapeutisches Verfahren unter Verwendung des erfindungsgemäßen Kollagenpeptids zur optischen und strukturellen Verbesserung der Haare, insbesondere zur Verbesserung der Haarqualität, Verminderung von Spliss und/oder Reduktion/Verzögerung von Haarausfall.

Offenbart (nicht erfindungsgemäß) wird die nicht-therapeutische Verwendung des erfindungsgemäßen Kollagenpeptids zur Steigerung der Mitochondrienzahl und/oder Mitochondrienaktivität.

Offenbart (nicht erfindungsgemäß) wird die nicht-therapeutische Verwendung des erfindungsgemäßen Kollagenpeptids zur Verbesserung der Ausdauerleistungsfähigkeit.

Offenbart (nicht erfindungsgemäß) wird die nicht-therapeutische Verwendung des erfindungsgemäßen Kollagenpeptids zur Verbesserung der mentalen Leistungsfähigkeit.

In bevorzugter Ausführungsform der vorliegenden Erfindung wird das erfindungsgemäße synthetische oder rekombinante Kollagenpeptid allein, das heißt ohne weitere Stoffe, zur Anwendung in einer der erfindungsgemäß vorgesehenen Anwendungen eingesetzt.

In einer weiteren Ausführungsform der vorliegenden Erfindung wird das erfindungsgemäße synthetische oder rekombinante Kollagenpeptid als das einzige biologische Wirksamkeit aufweisende Agens in einer erfindungsgemäß vorgesehenen Anwendung eingesetzt.

In einer weiteren bevorzugten Ausführungsform wird das erfindungsgemäße synthetische oder rekombinante Kollagenpeptid gemeinsam mit mindestens einem weiteren Agens, insbesondere einem weiteren biologisch wirkenden Agens in einer erfindungsgemäß vorgesehenen Anwendung eingesetzt.

Die vorliegende Erfindung betrifft auch eine pharmazeutische Zusammensetzung gemäß des Anspruchssatzes, umfassend ein erfindungsgemäßes Kollagenpeptid und mindestens einen pharmazeutisch akzeptablen Zusatzstoff, sowie die pharmazeutische Zusammensetzung zur Anwendung in einem Verfahren zur therapeutischen Behandlung des menschlichen oder tierischen Körpers, insbesondere zur Anwendung in mindestens einer der vorgenannten Indikationen. Demnach kann vorgesehen sein, das erfindungsgemäße Kollagenpeptid in Form einer pharmazeutischen Zusammensetzung zu verabreichen. Besonders vorteilhaft wird die erfindungsgemäße pharmazeutische Zusammensetzung beispielsweise im Formen von Tabletten, Lutschtabletten, Kautabletten, Kapseln, Beißkapseln, Dragees, Pastillen, Säften, Gels oder Salben verabreicht.

Die vorliegende Erfindung betrifft ferner ein Nahrungsergänzungsmittel gemäß des Anspruchssatzes, umfassend ein erfindungsgemäßes Kollagenpeptid und mindestens einen Nahrungsmittel-akzeptablen Zusatzstoff, sowie das Nahrungsergänzungsmittel zur Anwendung in einem Verfahren zur therapeutischen Behandlung des menschlichen oder tierischen Körpers, insbesondere zur Anwendung in mindestens einer der vorgenannten Indikationen. Demnach kann vorgesehen sein, das erfindungsgemäße Kollagenpeptid in Form eines Nahrungsergänzungsmittels zu verabreichen. Besonders vorteilhaft liegt das erfindungsgemäße Nahrungsergänzungsmittel als Tablette, Dragee, Pastille, Sachet, Lösung, Suspension oder Gel, zum Beispiel in einer Ampulle, als Granulat oder Pulver vor. Auf Grund seiner guten Löslichkeit kann das erfindungsgemäße Kollagenpeptid auch verschiedenen Getränken zugesetzt werden, ohne eine Trübung zu verursachen.

Gegenstand der vorliegenden Erfindung ist auch ein kosmetisches Produkt gemäß des Anspruchssatzes, umfassend ein erfindungsgemäßes Kollagenpeptid und mindestens einen hautverträglichen Zusatzstoff, sowie das kosmetische Produkt zur Anwendung in einem Verfahren zur therapeutischen Behandlung des menschlichen oder tierischen Körpers, insbesondere zur Anwendung in mindestens einer der vorgenannten Indikationen. Demgemäß kann es vorgesehen sein, das erfindungsgemäße Kollagenpeptid in Form einer kosmetischen Zusammensetzung zu verabreichen. Besonders vorteilhaft wird die erfindungsgemäße kosmetische Zusammensetzung beispielsweise im Formen von Lotionen, Salben, Cremes, Gelen, Puder, Spritzen oder Sprays verabreicht.

Gegenstand der Erfindung ist auch ein Lebensmittel oder Genussmittel gemäß des Anspruchssatzes, umfassend ein erfindungsgemäßes Kollagenpeptid, sowie das Lebensmittel oder Genussmittel zur Anwendung in einem Verfahren zur therapeutischen Behandlung des menschlichen oder tierischen Körpers, insbesondere zur Anwendung in mindestens einer der vorgenannten Indikationen. Gemäß einer bevorzugten Ausführungsform handelt es sich bei dem Lebensmittel oder Genussmittel um einem Schokoriegel, Proteinriegel, Cerealienriegel, Instantpulver zur Zubereitung von Getränken, Milch, Milchprodukte, zum Beispiel Joghurt, Molke oder Quark und Milchersatz, zum Beispiel Sojamilch, Reismilch, Mandelmilch und Kokosmilch (sog. Functional Food) oder ein Getränk, z.B. Erfrischungs- oder Fitnessgetränk.

Sofern das Kollagenpeptid gemäß einer bevorzugten Ausführungsform der Erfindung nicht als einziger physiologisch aktiver Bestandteil eines Produktes, insbesondere einer pharmazeutischen Zusammensetzung, eines Nahrungsergänzungsmittels, einer kosmetischen Zusammensetzung oder eines Lebensmittels oder Genussmittels, eingesetzt wird, kann es mit einer oder mehreren weiteren Komponenten kombiniert werden, die einen positiven Effekt auf die allgemeine Gesundheit aufweisen, insbesondere auf die Ausdauerleistungsfähigkeit. Solche Komponenten sind bevorzugt ausgewählt aus der Gruppe bestehend aus Vitamin C, Vitaminen der B-, D-, E- und K-Reihe, Omega-3-Fettsäuren, Omega-6-Fettsäuren, konjugierten Linolensäuren, Coffein und dessen Derivaten, Guaranäextrakt, Hagebuttenextrakt, Grünteeextrakt, Epigallocatechingallat, Kreatin, L-Carnitin, α-Liponsäure, N-Acetylcystein, NADH, D-Ribose, Magnesiumaspartat, Antioxidantien wie Anthocyane, Carotinoide, Flavonoide, Resveratrol, Glutathion und Superoxiddismutase (SOD), Cannabinoide wie Cannabidiol (CBD), Adaptogene wie *Rhodiola rosea, Panax ginseng, Withania somnifera,* Shiitake, *Ganoderma lucidum Lepidium meyenii,* Mineralstoffen wie Eisen, Magnesium, Calcium, Zink, Selen und Phosphor, sowie weiteren Proteinen, Hydrolysaten und Peptiden wie Soja-, Weizen- und Molkenprotein.

In weiteren bevorzugter Ausführungsform der vorliegenden Erfindung enthalten die erfindungsgemäßen Produkte, insbesondere die pharmazeutische Zusammensetzung, das Nahrungsergänzungsmittel, die kosmetische Zusammensetzung oder das Lebensmittel oder Genussmittel, neben dem erfindungsgemäßen Kollagenpeptid keine weiteren Proteine oder Peptide, insbesondere keine weiteren Kollagenpeptide.

In einer bevorzugten Ausführungsform der Erfindung wird das Kollagenpeptid in einer Menge von 1 bis 40 g pro Tag verabreicht, bevorzugt von 1 bis 30 g pro Tag, bevorzugt von 1 bis 20 g pro Tag, bevorzugt von 1 bis 15 g pro Tag, bevorzugt von 2,5 bis 30 g pro Tag, bevorzugt 2,5 bis 20 g pro Tag, bevorzugt 2,5 bis 15 g pro Tag, bevorzugt 2,5 bis 10 g pro Tag, bevorzugt 4 bis 15 g pro Tag, bevorzugt 4 bis 12 g pro Tag, weiter bevorzugt von 5 bis 25 g pro Tag, bevorzugt 5 bis 15 g pro Tag, bevorzugt 10 bis 25 g pro Tag, bevorzugt 12 bis 22 g pro Tag, bevorzugt 6 bis 15 g pro Tag, insbesondere von 2,5 bis 7,5 g pro Tag, bevorzugt 2,5 bis 5 g pro Tag.

Die vorliegende Erfindung betrifft auch Verfahren zur Prävention und/oder Behandlung der vorgenannten Indikationen, insbesondere der vorgenannten therapeutischen Indikationen, gemäß denen dem menschlichen oder tierischen Körper eine für den therapeutischen Zweck ausreichende Menge mindestens eines der erfindungsgemäße rekombinanten oder synthetisch erzeugten Peptide, gegebenenfalls mit einem Zusatzstoff, verabreicht wird.

Offenbart (nicht erfindungsgemäß) wird auch ein nicht-therapeutisches Verfahren zur Verbesserung der Muskelkraft, zur Vermehrung der Muskelmasse, zur Stimulation des Fettabbaus, zur Reduktion des Körpergewichtes, zum Erhalt und/oder zur Verbesserung der Knochengesundheit, zum Erhalt und/oder zur Verbesserung der Hautgesundheit, zum Erhalt und/oder zur Verbesserung der Darmgesundheit, zum Erhalt und/oder Verbesserung der Blutgefäßstruktur, zum Erhalt und oder Verbesserung der Gesundheit des Herzkreislaufsystems, zum Erhalt und/oder Verbesserung des Zahnhalteapparates, zum Erhalt und/oder zur Verbesserung der Gesundheit der Nägel und Haare eines menschlichen oder tierischen Körpers, zum Erhalt und/oder zur Steigerung der Mitochondrienzahl und/oder Mitochondrienaktivität, zum Erhalt und/oder Verbesserung der Ausdauerleistungsfähigkeit oder zum Erhalt und/oder zur Verbesserung der mentalen Leistungsfähigkeit, wobei dem menschlichen oder tierischen Körper mindestens ein erfindungsgemäßes Kollagenpeptid verabreicht wird.

Offenbart (nicht erfindungsgemäß) wird auch ein Verfahren zur Herstellung eines erfindungsgemäßen Kollagenpeptids, umfassend die Verfahrensschritte:
a) Bereitstellen eines Expressionssystems, das mindestens eine Expressionskassette aufweist, wobei die Expressionskassette mindestens eine Nukleotidsequenz aufweist, die ein Kollagenpeptid mit einem Molekulargewicht in einem Bereich von 0,18 bis 10 kDa codiert, insbesondere 0,18 bis 5,0 kDa, insbesondere 1,1 bis 5,0 kDa codiert,
b) Kultivieren des Expressionssystems unter Bedingungen, die die Expression des Kollagenpeptids ermöglichen, und
c) Gewinnen des erfindungsgemäßen Kollagenpeptids.

Das Verfahren zur Herstellung eines erfindungsgemäßen Kollagenpeptids, insbesondere eines synthetischen oder rekombinanten Kollagenpeptids zur therapeutischen Behandlung des menschlichen oder tierischen Körpers mit einem Molekulargewicht in einem Bereich von 0,18 bis 10,0 kDa, insbesondere 0,18 bis 5,0 kDa, insbesondere 1,1 bis 5,0 kDa, zeichnet sich insbesondere dadurch aus, dass ein genau definiertes, rekombinant hergestellten Kollagenpeptid mit einem Molekulargewicht in einem Bereich von 0,18 bis 10,0 kDa, insbesondere 0,18 bis 5,0 kDa, insbesondere 1,1 bis 5,0 kDa, gewonnen wird, welches sich, insbesondere aufgrund dessen biologischer Wirksamkeit, zur Anwendung in einem Verfahren zur therapeutischen Behandlung des menschlichen oder tierischen Körpers eignet.

Das erfindungsgemäß bereitgestellte Kollagenpeptid weist aufgrund seiner rekombinanten Herstellweise im Vergleich zu hydrolytisch aus natürlichen Quellen gewonnenen Kollagenpeptiden eine besonders hohe Reinheit auf. Es lässt sich dabei in den verschiedensten Expressionssystemen auch im industriellen Maßstab ohne unerwünschte Kontaminationen bereitstellen, wobei das erfindungsgemäße Kollagenpeptid gleichzeitig vorteilhafterweise eine biologische Wirksamkeit aufweist, insbesondere in Hinblick auf die Anwendungen in einem Verfahren zur therapeutischen Behandlung des menschlichen oder tierischen Körpers.

Die erfindungsgemäß gefundene biologische Wirksamkeit der erfindungsgemäßen Kollagenpeptide und damit verbunden deren Eignung zur Anwendung in einem Verfahren zur therapeutischen Behandlung des menschlichen oder tierischen Körpers, kommt vorteilhafterweise bereits den unmittelbar aus dem erfindungsgemäßen Verfahren gewonnenen Kollagenpeptiden zu, ohne dass es weiterer Verarbeitungsschritte bedarf. So zeigen sowohl die hydroxylierten als auch die nicht-hydroxylierten Kollagenpeptide gemäß der vorliegenden Erfindung eine biologische Wirksamkeit, insbesondere zumindest die gleiche biologische Wirksamkeit wie aus natürlichen Quellen gewonnene Kollagenpeptide des gleichen Molekulargewichts und/oder Gemische von aus natürlichen Quellen gewonnenen Kollagenpeptiden mit vergleichbarem durchschnittlichem Molekulargewicht. Besonders vorteilhaft ist hierbei, dass die erfindungsgemäßen Kollagenpeptide überraschenderweise selbst in nicht-hydroxylierter Form eine biologische Wirksamkeit aufweisen, bevorzugt die gleiche biologische Wirksamkeit wie aus natürlichen Quellen gewonnene hydroxylierte Kollagenpeptide des gleichen Molekulargewichts und/oder Gemische von aus natürlichen Quellen gewonnenen hydroxylierten Kollagenpeptiden mit vergleichbarem durchschnittlichem Molekulargewicht, aufweisen, besonders bevorzugt eine bessere biologische Wirksamkeit wie aus natürlichen Quellen gewonnene hydroxylierte Kollagenpeptide des gleichen Molekulargewichts und/oder Gemische von aus natürlichen Quellen gewonnenen hydroxylierten Kollagenpeptiden mit vergleichbarem durchschnittlichem Molekulargewicht aufweisen.

Bevorzugt zeigen die hydroxylierten als auch die nicht-hydroxylierten Kollagenpeptide gemäß der vorliegenden Erfindung in Osteoblasten, Fibroblasten und/oder Chondrocyten, bevorzugt in Osteoblasten, bevorzugt in Fibroblasten, bevorzugt in Chondrocyten, bevorzugt in Osteoblasten und Fibroblasten, bevorzugt in Osteoblasten und Chondrocyten, bevorzugt in Fibroblasten und Chondrocyten, eine biologische Wirksamkeit, bevorzugt zumindest die gleiche biologische Wirksamkeit wie aus natürlichen Quellen gewonnene Kollagenpeptide des gleichen Molekulargewichts und/oder Gemische von aus natürlichen Quellen gewonnenen Kollagenpeptiden mit vergleichbarem durchschnittlichem Molekulargewicht, besonders bevorzugt eine bessere biologische Wirksamkeit wie aus natürlichen Quellen gewonnene Kollagenpeptide des gleichen Molekulargewichts und/oder Gemische von aus natürlichen Quellen gewonnenen Kollagenpeptiden mit vergleichbarem durchschnittlichem Molekulargewicht.

Die erfindungsgemäß gefundene und den synthetischen oder rekombinanten Kollagenpeptiden, insbesondere den synthetisch oder rekombinant hergestellten Kollagenpeptiden, der vorliegenden Erfindung zukommende biologische Wirksamkeit, insbesondere in Bindegewebe, insbesondere in Bindegewebe der Haut, des Darms, der Blutgefäße und des Zahnhalteapparates, Gehirnhaut, Sehnen, Bändern, Knorpel und/oder Knochen, lässt sich insbesondere anhand der in den Beispielen 3 bis 6 dargestellten in vitro-Tests zur Stimulation der Synthese von extrazellulären Matrixproteinen in Osteoblasten, Fibroblasten und Chondrocyten feststellen.

In bevorzugter Ausführungsform weisen die erfindungsgemäßen synthetischen oder rekombinanten Kollagenpeptide, insbesondere die synthetisch oder rekombinant hergestellten Kollagenpeptide, insbesondere die synthetisch oder rekombinant hergestellten hydroxylierten Kollagenpeptide, insbesondere die synthetisch oder rekombinant hergestellten nicht-hydroxylierten Kollagenpeptide, der vorliegenden Erfindung eine biologische Wirksamkeit auf, insbesondere weisen sie in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine biologische Wirksamkeit auf.

Bevorzugt weisen die erfindungsgemäßen synthetischen oder rekombinanten Kollagenpeptide, insbesondere die synthetisch oder rekombinant hergestellten Kollagenpeptide, insbesondere die synthetisch oder rekombinant hergestellten hydroxylierten Kollagenpeptide, insbesondere die synthetisch oder rekombinant hergestellten nicht-hydroxylierten Kollagenpeptide, der vorliegenden Erfindung die gleiche biologische Wirksamkeit auf wie aus natürlichen Quellen isolierte Kollagenpeptide des gleichen Molekulargewichts, insbesondere weisen sie in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten die gleiche biologische Wirksamkeit auf wie aus natürlichen Quellen isolierte Kollagenpeptide des gleichen Molekulargewichts.

Besonders bevorzugt weisen die erfindungsgemäßen synthetischen oder rekombinanten Kollagenpeptide, insbesondere die synthetisch oder rekombinant hergestellten Kollagenpeptide, insbesondere die synthetisch oder rekombinant hergestellten hydroxylierten Kollagenpeptide, insbesondere die synthetisch oder rekombinant hergestellten nicht-hydroxylierten Kollagenpeptide, der vorliegenden Erfindung die gleiche biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht, insbesondere weisen sie in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten die gleiche biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht.

Besonders bevorzugt weisen die erfindungsgemäßen synthetischen oder rekombinanten Kollagenpeptide, insbesondere die synthetisch oder rekombinant hergestellten Kollagenpeptide, insbesondere die synthetisch oder rekombinant hergestellten hydroxylierten Kollagenpeptide, insbesondere die synthetisch oder rekombinant hergestellten nicht-hydroxylierten Kollagenpeptide, der vorliegenden Erfindung eine bessere biologische Wirksamkeit auf als aus natürlichen Quellen isolierte Kollagenpeptide des gleichen Molekulargewichts, insbesondere weisen sie in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine bessere biologische Wirksamkeit auf als aus natürlichen Quellen isolierte Kollagenpeptide des gleichen Molekulargewichts.

Besonders bevorzugt weisen die erfindungsgemäßen synthetischen oder rekombinanten Kollagenpeptide, insbesondere die synthetisch oder rekombinant hergestellten Kollagenpeptide, insbesondere die synthetisch oder rekombinant hergestellten hydroxylierten Kollagenpeptide, insbesondere die synthetisch oder rekombinant hergestellten nicht-hydroxylierten Kollagenpeptide, der vorliegenden Erfindung eine bessere biologische Wirksamkeit auf als aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht, insbesondere weise sie in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine bessere biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht.

Das in Schritt a) bereitgestellte Expressionssystem kann eine Wirtszelle sein, insbesondere eine prokaryotische oder eukaryotische Zelle.

Das Expressionssystem kann eine Wirtszelle sein ausgewählt aus der Gruppe bestehend aus Bakterienzelle, Hefezelle, Pilzzelle, Säugetierzelle, Insektenzelle und Pflanzenzelle.

Das Expressionssystem, insbesondere die Wirtszelle, kann eine Bakterienzelle sein, insbesondere der Arten *Escherichia coli* oder *Bacillus subtilis.*

Das Expressionssystem, insbesondere die Wirtszelle, kann eine Hefezelle sein, insbesondere der Arten *Saccharomyces cerevisiae, Pichia pastoris* oder *Ogataea angusta (Hansenula polymorpha).*

Das Expressionssystem, insbesondere die Wirtszelle, kann eine Pilzzelle sein, insbesondere der Art *Aspergillus niger.*

Das Expressionssystem, insbesondere die Wirtzelle, kann eine Säugetierzelle sein, insbesondere eine CHO-Zelle, eine HeLa-Zelle oder eine HEK293-Zelle.

Das Expressionssystem, insbesondere die Wirtszelle, kann eine Insektenzelle sein, insbesondere eine Sf-9, Sf-21 oder Tn-5 Zelle.

Das Expressionssystem, insbesondere die Wirtszelle, kann eine Pflanzenzelle sein, insbesondere eine Mais- oder Tabakzelle.

Das in Schritt a) bereitgestellte Expressionssystem kann eine Wirtszelle sein, die zur Hydroxylierung von Prolin-, Lysin- oder Prolin- und Lysinresten des exprimierten Kollagenpeptids befähigt ist. Das in Schritt a) bereitgestellte Expressionssystem kann eine Wirtszelle sein, die zur Hydroxylierung von Prolin-, Lysin- oder Prolin- und Lysinresten des exprimierten Kollagenpeptids befähigt ist.

Das in Schritt a) bereitgestellte Expressionssystem kann ein Expressionssystem sein, das Prolylhydroxylase- und/oder Lysylhydroxylase-Aktivität aufweist. Das in Schritt a) bereitgestellte Expressionssystem kann eine Wirtszelle sein, die Prolylhydroxylase- und/oder Lysylhydroxylase-Aktivität aufweist.

Das in Schritt a) bereitgestellte Expressionssystem kann eine Wirtszelle sein, die mindestens eine Expressionskassette aufweist, die eine Prolyl-4-hydroxylase-codierende Polynukleotidsequenz umfasst. Das in Schritt a) bereitgestellte Expressionssystem kann eine Wirtszelle sein, die mindestens eine Expressionskassette aufweist, die eine Prolyl-4-hydroxylase-codierende Polynukleotidsequenz umfasst, so dass in Verfahrensschritt c) ein in vivo hydroxyliertes Kollagenpeptid gewonnen wird.

Das in Schritt a) bereitgestellte Expressionssystem kann eine Wirtszelle sein, die mindestens eine Expressionskassette aufweist, die eine Lysylhydroxylase-codierende Polynukleotidsequenz umfasst. Das in Schritt a) bereitgestellte Expressionssystem kann eine Wirtszelle sein, die mindestens eine Expressionskassette aufweist, die eine Lysylhydroxylase-codierende Polynukleotidsequenz umfasst, so dass in Verfahrensschritt c) ein in vivo hydroxyliertes Kollagenpeptid gewonnen wird.

Das in Schritt a) bereitgestellte Expressionssystem kann eine Wirtszelle sein, die mindestens eine Expressionskassette aufweist, die eine Prolyl-4-hydroxylase-codierende Polynukleotidsequenz umfasst und mindestens eine Expressionskassette, die eine Lysylhydroxylase-codierende Polynukleotidsequenz umfasst. Das in Schritt a) bereitgestellte Expressionssystem kann eine Wirtszelle sein, die mindestens eine Expressionskassette aufweist, die eine Prolyl-4-hydroxylase-codierende Polynukleotidsequenz umfasst und mindestens eine Expressionskassette, die eine Lysylhydroxylase-codierende Polynukleotidsequenz umfasst, so dass in Verfahrensschritt c) ein in vivo hydroxyliertes Kollagenpeptid gewonnen wird.

Offenbart (nicht erfindungsgemäß) wird auch ein Verfahren zur Herstellung eines erfindungsgemäßen Kollagenpeptids, insbesondere eines in vivo hydroxylierten Kollagenpeptids, umfassend die Verfahrensschritte
a) Bereitstellen eines Expressionssystems, das mindestens eine Expressionskassette aufweist, wobei die Expressionskassette mindestens eine Nukleotidsequenz aufweist, die ein Kollagenpeptid mit einem Molekulargewicht in einem Bereich von 0,18 bis 10,0 kDa codiert, insbesondere 0,18 bis 5,0 kDa, insbesondere 1,1 bis 5,0 kDa codiert, und wobei das Expressionssystem zur Hydroxylierung von Prolin-, Lysin- oder Prolin- und Lysinresten des exprimierten Kollagenpeptids befähigt ist,
b) Kultivieren des Expressionssystems unter Bedingungen, die die Expression und Hydroxylierung des Kollagenpeptids ermöglichen,
c) Gewinnen des erfindungsgemäßen Kollagenpeptids, insbesondere des in vivo hydroxylierten Kollagenpeptids.

Mit Hilfe des vorgenannten Verfahrens ist es somit möglich, ein in vivo hydroxyliertes rekombinant hergestelltes Kollagenpeptid mit einem spezifischen Molekulargewicht in einem Bereich von 0,18 bis 10,0 kDa, insbesondere 0,18 bis 5,0 kDa, insbesondere 1,1 bis 5,0 kDa, zu erhalten, welches sich je nach verwendetem zellbasierten Expressionssystem durch ein spezifisches Muster an posttranslationalen Modifikationen, insbesondere Hydroxylierungen und Glykosylierungen auszeichnen. Auf diese Weise ist es vorteilhafterweise insbesondere möglich, direkt, das heißt ohne die Notwendigkeit einer nachträglichen Modifizierung, ein Kollagenpeptid mit gewünschter biologischer Wirksamkeit, insbesondere ein rekombinant hergestelltes Kollagenpeptid zur Anwendung in einem Verfahren zur therapeutischen Behandlung des menschlichen oder tierischen Körpers, zu erhalten.

In bevorzugter Ausführungsform weist das hergestellte rekombinante in vivo hydroxylierte Kollagenpeptid eine biologische Wirksamkeit auf, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine biologische Wirksamkeit auf.

Bevorzugt weist das hergestellte rekombinante in vivo hydroxylierte Kollagenpeptid die gleiche biologische Wirksamkeit auf wie aus natürlichen Quellen isolierte Kollagenpeptide des gleichen Molekulargewichts, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten die gleiche biologische Wirksamkeit auf wie aus natürlichen Quellen isolierte Kollagenpeptide des gleichen Molekulargewichts.

Besonders bevorzugt weist das hergestellte rekombinante in vivo hydroxylierte Kollagenpeptid die gleiche biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten die gleiche biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht.

Besonders bevorzugt weist das hergestellte rekombinante in vivo hydroxylierte Kollagenpeptid eine bessere biologische Wirksamkeit auf als aus natürlichen Quellen isolierte Kollagenpeptide des gleichen Molekulargewichts, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine bessere biologische Wirksamkeit auf als aus natürlichen Quellen isolierte Kollagenpeptide des gleichen Molekulargewichts.

Besonders bevorzugt weist das hergestellte rekombinante in vivo hydroxylierte Kollagenpeptid eine bessere biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine bessere biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht.

Das in Schritt a) bereitgestellte Expressionssystem kann ein Expressionssystem sein, das nicht dazu befähig ist, eine Hydroxylierung von Prolin-, Lysin- oder Prolin- und Lysinresten des exprimierten Kollagenpeptids zu bewirken, insbesondere weist das in Schritt a) bereitgestellte Expressionssystem keine Prolylhydroxylase- und Lysylhydroxylase-Aktivität auf.

Offenbart (nicht erfindungsgemäß) wird auch ein Verfahren zur Herstellung eines erfindungsgemäßen Kollagenpeptids, insbesondere eines nicht-hydroxylierten Kollagenpeptids, umfassend die Verfahrensschritte
a) Bereitstellen eines Expressionssystems, das mindestens eine Expressionskassette aufweist, wobei die Expressionskassette mindestens eine Nukleotidsequenz aufweist, die ein Kollagenpeptid mit einem Molekulargewicht in einem Bereich von 0,18 bis 10,0 kDa codiert, insbesondere 0,18 bis 5,0 kDa, insbesondere 1,1 bis 5,0 kDa codiert, und wobei das Expressionssystem nicht zur Hydroxylierung von Prolin-, Lysin- oder Prolin- und Lysinresten des exprimierten Kollagenpeptids befähigt ist,
b) Kultivieren des Expressionssystems unter Bedingungen, die die Expression des Kollagenpeptids ermöglichen,
c) Gewinnen des erfindungsgemäßen Kollagenpeptids, insbesondere des nicht-hydroxylierten Kollagenpeptids.

In bevorzugter Ausführungsform weist das hergestellte rekombinante nicht-hydroxylierte Kollagenpeptid eine biologische Wirksamkeit auf, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine biologische Wirksamkeit auf.

Bevorzugt weist das hergestellte rekombinante nicht-hydroxylierte Kollagenpeptid die gleiche biologische Wirksamkeit auf wie aus natürlichen Quellen isolierte Kollagenpeptide des gleichen Molekulargewichts, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten die gleiche biologische Wirksamkeit auf wie aus natürlichen Quellen isolierte Kollagenpeptide des gleichen Molekulargewichts.

Besonders bevorzugt weist das hergestellte rekombinante nicht-hydroxylierte Kollagenpeptid die gleiche biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten die gleiche biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht.

Besonders bevorzugt weist das hergestellte rekombinante nicht-hydroxylierte Kollagenpeptid eine bessere biologische Wirksamkeit auf als aus natürlichen Quellen isolierte Kollagenpeptide des gleichen Molekulargewichts, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine bessere biologische Wirksamkeit auf als aus natürlichen Quellen isolierte Kollagenpeptide des gleichen Molekulargewichts.

Besonders bevorzugt weist das hergestellte rekombinante nicht-hydroxylierte Kollagenpeptid eine bessere biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht, insbesondere weist es in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten eine bessere biologische Wirksamkeit auf wie aus natürlichen Quellen isolierte Gemische an Kollagenpeptiden, insbesondere wie aus natürlichen Quellen isolierte Gemische an Kollagenpeptiden mit vergleichbarem durchschnittlichem Molekulargewicht.

Gemäß einer bevorzugten Ausführungsform der vorliegenden Offenbarung ist die mindestens eine Nukleotidsequenz der mindestens einen Expressionskassette codon-optimiert, das heißt, dass diejenigen Codons in der Nukleotidsequenz, die vom Translationssystem des bereitgestellten Expressionssystems, insbesondere des bereitgestellten zellbasierten Expressionssystems, insbesondere der bereitgestellten Wirtszelle, nicht oder nicht bevorzugt genutzt werden, durch solche ersetzt werden, die vom Translationssystem des bereitgestellten Expressionssystems, insbesondere des bereitgestellten zellbasierten Expressionssystems, insbesondere der bereitgestellten Wirtszelle, bevorzugt verwendet werden ohne dadurch die Aminosäuresequenz des codierten Peptids oder Proteins zu verändern.

In einer bevorzugten Ausführungsform der vorliegenden Offenbarung ist das durch die Nukleotidsequenz codierte Kollagenpeptid ein Kollagenpeptid eines Wirbeltiers, insbesondere eines Säugetiers, zum Beispiel des Menschen oder eines nicht-menschlichen Säugetiers, zum Beispiel Pferd, Esel, Känguru, Schaf, Nagetier, Schwein oder Rind, eines Vogels, zum Beispiel Huhn, eines Fisches, einer Amphibie, eines Reptils oder eines wirbellosen Tieres, zum Beispiel Qualle.

Gemäß einer bevorzugten Ausführungsform der vorliegenden Erfindung codiert die mindestens eine Nukleotidsequenz ein Kollagenpeptid mit einem Molekulargewicht in einem Bereich von 0,18 bis 5,0 kDa, bevorzugt 0,2 bis 5,0 kDa, bevorzugt 0,3 bis 5,0 kDa, bevorzugt 0,4 bis 5,0 kDa, bevorzugt 0,5 bis 5,0 kDa, bevorzugt 0,6 bis 5,0 kDa, bevorzugt 0,7 bis 5,0 kDa, bevorzugt 0,8 bis 5,0 kDa, bevorzugt 0,9 bis 5,0 kDa, bevorzugt 1,0 bis 5,0 kDa, bevorzugt 1,1 bis 5,0 kDa, bevorzugt 1,2 bis 5,0 kDa, bevorzugt 1,3 bis 5,0 kDa, bevorzugt 1,4 bis 5,0 kDa, bevorzugt 1,5 bis 5,0 kDa, bevorzugt 1,6 bis 5,0 kDa, bevorzugt 1,7 bis 5,0 kDa, bevorzugt 1,8 bis 5,0 kDa, bevorzugt 1,9 bis 5,0 kDa, bevorzugt 2,0 bis 5,0 kDa, bevorzugt 2,1 bis 4,9 kDa, bevorzugt 2,2 bis 4,8 kDa, bevorzugt 2,3 bis 4,7 kDa, bevorzugt 2,4 bis 4,6 kDa, bevorzugt 2,5 bis 4,5 kDa, bevorzugt 1,2 bis 3,2, bevorzugt 1,3 bis 3,0 kDa, bevorzugt 1,5 bis 3,0 kDa, bevorzugt 1,8 bis 3,0 kDa, bevorzugt 2,0 bis 3,0 kDa.

In einer bevorzugten Ausführungsform der vorliegenden Erfindung ist das mit einem der vorgenannten Verfahren hergestellte Kollagenpeptid gemäß des Anspruchssatzes ein nicht-hydroxyliertes, partial hydroxyliertes oder vollständig hydroxyliertes Kollagenpeptid, bevorzugt ein nicht-hydroxyliertes Kollagenpeptid, bevorzugt ein partial hydroxyliertes Kollagenpeptid, bevorzugt ein vollständig hydroxyliertes Kollagenpeptid.

In einer bevorzugten Ausführungsform der vorliegenden Erfindung ist das mit einem der Verfahren hergestellte Kollagenpeptid gemäß des Anspruchssatzes ein glykosyliertes Kollagenpeptid. Bevorzugt ist das Kollagenpeptid in vivo glykosyliert, bevorzugt ex vivo glykosyliert.

In einer weiteren bevorzugten Ausführungsform der vorliegenden Erfindung ist das mit einem der Verfahren hergestellte Kollagenpeptid ein nicht-glykosyliertes Kollagenpeptid.

Offenbart (nicht erfindungsgemäß) wird auch ein Verfahren zur Herstellung eines erfindungsgemäßen Kollagenpeptids, wobei die mindestens eine Expressionskassette des Expressionssystems mehrere, bevorzugt mindestens zwei, bevorzugt mindestens drei, bevorzugt mindestens vier, bevorzugt mindestens fünf, aneinandergereihte Nukleotidsequenzen aufweist, die jeweils ein Kollagenpeptid mit einem Molekulargewicht in einem Bereich von 0,18 bis 10,0 kDa codiert, insbesondere 0,18 bis 5,0 kDa, insbesondere 1,1 bis 5,0 kDa codieren.

So wird durch das Expressionssystem zunächst ein Vorläufer-Kollagenpeptid synthetisiert, aus welchem anschließend durch Spaltung, insbesondere enzymatische Spaltung, erfindungsgemäße Kollagenpeptide erhalten werden können.

Die Größe des Vorläufer-Kollagenpeptids kann 2 bis 100 kDa, bevorzugt 3 bis 80 kDa, bevorzugt 4 bis 60 kDa, bevorzugt 5 bis 50 kDa, bevorzugt 10 bis 45 kDa betragen. Das Vorläufer-Kollagenpeptid kann 2 bis 40 erfindungsgemäße Kollagenpeptide, bevorzugt 3 bis 30 erfindungsgemäße Kollagenpeptide, bevorzugt 5 bis 20 erfindungsgemäße Kollagenpeptide umfassen.

Die mehrere, bevorzugt mindestens zwei, bevorzugt mindestens drei, bevorzugt mindestens vier, bevorzugt mindestens fünf, jeweils ein Kollagenpeptid mit einem Molekulargewicht in einem Bereich von 0,18 bis 10,0 kDa, insbesondere 0,18 bis 5,0 kDa, insbesondere 1,1 bis 5,0 kDa codierenden aneinandergereihte Nukleotidsequenzen können durch Sequenzen getrennt sein, die eine Spaltung, insbesondere eine enzymatische Spaltung, des in Schritt b) exprimierten Kollagenpeptids in mehrere, mindestens zwei, bevorzugt mindestens drei, mindestens vier, mindestens fünf, erfindungsgemäße Kollagenpeptide, erlauben.

Zwischen den Schritten b) und c) kann ein Schritt des Spaltens des in Schritt b) exprimierten Kollagenpeptids zum Erhalt von erfindungsgemäßen Kollagenpeptiden erfolgen, insbesondere Kollagenpeptiden mit einem Molekulargewicht in einem Bereich von 0,18 bis 10,0 kDa, insbesondere 0,18 bis 5,0 kDa, insbesondere 1,1 bis 5,0 kDa. Demnach wird durch das in Schritt a) bereitgestellte Expressionssystem in Schritt b) ein Kollagenpeptid exprimiert, das anschließend gespalten wird, sodass mehrere, mindestens zwei, mindestens drei, mindestens vier, mindestens fünf, erfindungsgemäße Kollagenpeptide erhalten werden.

Es kann sich bei den mehreren, bevorzugt mindestens zwei, bevorzugt mindestens drei, bevorzugt mindestens vier, bevorzugt mindestens fünf, aneinandergereihten Nukleotidsequenzen um unterschiedliche Nukleotidsequenzen handeln. Es kann sich bei den mehreren, bevorzugt mindestens zwei, bevorzugt mindestens drei, bevorzugt mindestens vier, bevorzugt mindestens fünf, aneinandergereihten Nukleotidsequenzen um identische Nukleotidsequenzen handeln.

Die Spaltung, insbesondere die enzymatische Spaltung, des in Schritt b) exprimierten Kollagenpeptids, insbesondere des Vorläufer-Kollagenpeptids, kann mittels neutraler, alkalischer oder saurer Proteasen erfolgen.

Die einzelnen erfindungsgemäße Kollagenpeptide des exprimierten Kollagenpeptids, insbesondere des Vorläufer-Kollagenpeptids, können durch spezifische Erkennungssequenzen voneinander getrennt sein.

Die spezifischen Erkennungssequenzen können ausgewählt aus der Gruppe bestehend aus Faktor Xa (Ile-(Glu/Asp)-Gly-Arg), TEV (Glu-Asn-Leu-Tyr-Phe-Gln-(Gly/Ser)), Thrombin (Leu-Val-Pro-Arg-Gly-Ser), Trypsin-Erkennungssequenz, Papain-Erkennungssequenz sein.

In bevorzugter Ausführungsform wird gemäß der vorliegenden Erfindung das Kollagenpeptid lokal, insbesondere topisch, oder systemisch, insbesondere enteral, bevorzugt oral, oder parenteral, verabreicht.

Unter dem Begriff "biologische Wirksamkeit" wird erfindungsgemäß bevorzugt die Fähigkeit der erfindungsgemäßen Kollagenpeptide zur Stimulation der Synthese von extrazellulären Matrixproteinen, insbesondere der Synthese von Kollagen, Proteoglykanen und/oder Elastin, in Zellen, insbesondere Osteoblasten, Fibroblasten und/oder Chondrocyten, bezeichnet. Dabei liegt eine "biologische Wirksamkeit" erfindungsgemäß bevorzugt dann vor, wenn es durch die Inkubation von Zellen, insbesondere Osteoblasten, Fibroblasten und/oder Chondrocyten, mit den erfindungsgemäßen Kollagenpeptiden zur einer Stimulierung der Synthese von extrazellulären Matrixproteinen, insbesondere der Synthese von Kollagen, Proteoglykanen und/oder Elastin, vorzugsweise messbar in mindestens einem, bevorzugt mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten, im Vergleich zu unbehandelten Zellen und/oder mit einem biologisch nicht wirksamen Agens behandelten Zellen kommt, insbesondere unbehandelten Osteoblasten, Fibroblasten und/oder Chondrocyten, oder mit einem biologisch nicht wirksamen Agens behandelten Osteoblasten, Fibroblasten und/oder Chondrocyten. In einer bevorzugten Ausführungsform der vorliegenden Erfindung wird unter einem "biologisch nicht wirksamen Agens" 160 Bloom Gelatine aus Schweineschwarten oder eine 260 Bloom Gelatine aus Rinderspalt verstanden.

Unter einer "gleichen biologischen Wirksamkeit" wird erfindungsgemäß bevorzugt verstanden, dass die erfindungsgemäßen Kollagenpeptide eine Stimulation der Synthese von extrazellulären Matrixproteinen, insbesondere der Synthese von Kollagen, Proteoglykanen und/oder Elastin, in Zellen, insbesondere Osteoblasten, Fibroblasten und/oder Chondrocyten, bewirken, die mit der Stimulation von Zellen, insbesondere Osteoblasten, Fibroblasten und/oder Chondrocyten, vergleichbar ist, die bei Inkubation mit einem Gemisch von aus natürlichen Quellen gewonnenen Kollagenpeptiden, insbesondere bei Inkubation mit einem Gemisch von aus natürlichen Quellen gewonnenen Kollagenpeptiden mit vergleichbarem durchschnittlichem Molekulargewicht, insbesondere mit Verisol, hergestellt gemäß EP 2640352 B1, Fortigel, hergestellt gemäß WO 2010/149596 oder Fortibone, hergestellt gemäß WO 2014/072235 (EP 2916855 B1), gemessen werden kann. Eine "vergleichbare Stimulation" bezeichnet erfindungsgemäß bevorzugt eine Stimulation der Synthese von extrazellulären Matrixproteinen, insbesondere der Synthese von Kollagen, Proteoglykanen und/oder Elastin, in Zellen, insbesondere Osteoblasten, Fibroblasten und/oder Chondrocyten, vorzugsweise messbar in mindestens einem, bevorzugt mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten, die um höchstens 2%, bevorzugt höchstens 1,5%, bevorzugt höchstens 1% abweicht von der Stimulation der Synthese von extrazellulären Matrixproteinen, insbesondere der Synthese von Kollagen, Proteoglykanen und/oder Elastin, in Zellen, insbesondere Osteoblasten, Fibroblasten und/oder Chondrocyten, die durch Inkubation der Zellen mit einem Gemisch von aus natürlichen Quellen gewonnenen Kollagenpeptiden, insbesondere durch Inkubation der Zellen mit einem Gemisch von aus natürlichen Quellen gewonnenen Kollagenpeptiden mit vergleichbarem durchschnittlichem Molekulargewicht, insbesondere mit Verisol, hergestellt gemäß EP 2640352 B1, Fortigel, hergestellt gemäß WO 2010/149596 oder Fortibone, hergestellt gemäß WO 2014/072235 (EP 2916855 B1), bewirkt wird.

Unter dem Begriff "bessere biologische Wirksamkeit" wird erfindungsgemäß bevorzugt verstanden, dass die erfindungsgemäßen Kollagenpeptide eine stärkere Stimulation der Synthese von extrazellulären Matrixproteinen, insbesondere der Synthese von Kollagen, Proteoglykanen und/oder Elastin, in Zellen, insbesondere Osteoblasten, Fibroblasten und/oder Chondrocyten, bewirken, als ein Gemisch von aus natürlichen Quellen gewonnenen Kollagenpeptiden, insbesondere ein Gemisch von aus natürlichen Quellen gewonnenen Kollagenpeptiden mit vergleichbarem durchschnittlichem Molekulargewicht, insbesondere mit Verisol, hergestellt gemäß EP 2640352 B1, Fortigel, hergestellt gemäß WO 2010/149596 oder Fortibone, hergestellt gemäß WO 2014/072235 (EP 2916855 B1). Erfindungsgemäß bevorzugt wird unter einer "besseren biologischen Wirksamkeit" eine Steigerung der Stimulation der Synthese von extrazellulären Matrixproteinen, insbesondere der Synthese von Kollagen, Proteoglykanen und/oder Elastin, in Zellen, insbesondere Osteoblasten, Fibroblasten und/oder Chondrocyten, durch die erfindungsgemäßen Kollagenpeptide um mehr als 2%, bevorzugt mindestens 3%, bevorzugt mindestens 4%, bevorzugt mindestens 5%, im Vergleich zur Stimulation der Synthese von extrazellulären Matrixproteinen, insbesondere der Synthese von Kollagen, Proteoglykanen und/oder Elastin, in Zellen, insbesondere Osteoblasten, Fibroblasten und/oder Chondrocyten, durch ein Gemisch von aus natürlichen Quellen gewonnenen Kollagenpeptiden, insbesondere mit Verisol, hergestellt gemäß EP 2640352 B1, Fortigel, hergestellt gemäß WO 2010/149596 oder Fortibone, hergestellt gemäß WO 2014/072235 (EP 2916855 B1), verstanden, wobei die Steigerung der Synthese in mindestens einem, bevorzugt mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten, messbar ist.

Eine "biologische Wirksamkeit", eine "gleiche biologische Wirksamkeit" oder eine "bessere biologische Wirksamkeit" kann erfindungsgemäß demgemäß dann vorliegen, wenn die erfindungsgemäßen Kollagenpeptide zur Stimulation der Synthese von einem extrazellulären Matrixprotein in der Lage sind, vorzugsweise dann, wenn zumindest die Synthese eines der extrazellulären Matrixproteine Kollagen, Proteoglykan oder Elastin, vorzugsweise von zwei oder drei dieser extrazellulären Matrixproteine stimuliert wird und zwar in Zellen, vorzugsweise in zumindest einem der Zelltypen Osteoblasten, Fibroblasten oder Chondrocyten, vorzugsweise in zwei oder drei dieser Zelltypen. Demgemäß kann eine biologische Wirksamkeit in einer Ausführungsform der vorliegenden Erfindung auch dann vorliegen, wenn lediglich die Synthese eines extrazellulären Matrixproteins stimuliert wird, insbesondere beispielsweise ein extrazelluläres Matrixprotein ausgewählt aus der Gruppe bestehend aus Kollagen, Proteoglykan und Elastin, während für andere extrazellulären Matrixproteine keine Stimulation der Synthese in den betreffenden Zellen festgestellt wird. Ebenso kann eine biologische Wirksamkeit, beziehungsweise eine gleiche oder bessere biologische Wirksamkeit vorliegen, wenn in lediglich einem Zelltyp, insbesondere einem Zelltyp ausgewählt aus der Gruppe bestehend aus Osteoblasten, Fibroblasten und Chondrocyten, eine Stimulation der Synthese mindestens eines extrazellulären Matrixproteins festgestellt wird, selbst wenn in anderen Zelltypen eine solche Stimulation nicht auftritt. Bevorzugt ist vorgesehen, dass die Synthese von mehr als einem extrazellulären Matrixprotein, insbesondere von einem oder mehreren der explizit genannten extrazellulären Matrixproteine ausgewählt aus der Gruppe bestehend aus Kollagen, Proteoglykan und Elastin, in Zellen, insbesondere in mindestens einem der Zelltypen Osteoblasten, Fibroblasten oder Chondrocyten, insbesondere in zwei oder drei dieser Zelltypen, stimuliert wird.

Gemäß der vorliegenden Erfindung kann eine "biologische Wirksamkeit", eine "gleiche biologische Wirksamkeit" oder eine "bessere biologische Wirksamkeit" der erfindungsgemäß Kollagenpeptide in Osteoblasten nach Inkubation der Zellen mit den erfindungsgemäßen Kollagenpeptiden durch Bestimmung der Expression der mRNA der entsprechenden extrazellulären Matrixproteine, insbesondere ausgewählt aus der Gruppe bestehend aus Kollagen, Proteoglykan und Elastin, im Vergleich zur Expression der mRNA der entsprechenden extrazellulären Matrixproteine, insbesondere ausgewählt aus der Gruppe bestehend aus Kollagen, Proteoglykan und Elastin, in geeigneten Kontrollen mittels Realtime-PCR bestimmt werden, insbesondere durch den in Beispiel 3 aufgeführten in vitro-Test.

Eine "biologische Wirksamkeit", eine "gleiche biologische Wirksamkeit" oder eine "bessere biologische Wirksamkeit" der erfindungsgemäß Kollagenpeptide in Fibroblasten kann gemäß der vorliegenden Erfindung nach Inkubation der Zellen mit den erfindungsgemäßen Kollagenpeptiden durch Bestimmung der Expression der mRNA der entsprechenden extrazellulären Matrixproteine, insbesondere ausgewählt aus der Gruppe bestehend aus Kollagen, Biglykan und Versican, im Vergleich zur Expression der mRNA der entsprechenden extrazellulären Matrixproteine, insbesondere ausgewählt aus der Gruppe bestehend aus Kollagen, Biglykan und Versican, in geeigneten Kontrollen mittels Realtime-PCR nachgewiesen werden, insbesondere durch den in Beispiel 4 aufgeführten in vitro-Test.

Gemäß der vorliegenden Erfindung kann eine "biologische Wirksamkeit", eine "gleiche biologische Wirksamkeit" oder eine "bessere biologische Wirksamkeit" der erfindungsgemäß Kollagenpeptide in Chondrocyten nach Inkubation der Zellen mit den erfindungsgemäßen Kollagenpeptiden durch radioaktive Markierung und Detektion der Menge an synthetisiertem radioaktiv markiertem Kollagen und/oder durch Alcianblau-Färbung und photometrischer Bestimmung der Glykosaminoglykane (GAG) von synthetisierten Proteoglykanen jeweils im Vergleich zu geeigneten Kontrollen bestimmt werden, insbesondere durch die in Beispiel 5 aufgeführten in vitro-Tests.

Eine "biologische Wirksamkeit", eine "gleiche biologische Wirksamkeit" oder eine "bessere biologische Wirksamkeit" der erfindungsgemäß Kollagenpeptide in Osteoblasten, Fibroblasten und Chondrocyten, insbesondere in Fibroblasten und Chondrocyten, kann gemäß der vorliegenden Erfindung nach Inkubation der Zellen mit den erfindungsgemäßen Kollagenpeptiden durch Bestimmung der Menge an synthetisierten extrazellulären Matrixproteinen, insbesondere ausgewählt aus der Gruppe bestehend aus Kollagen, Proteoglykan und Elastin, besonders bevorzugt ausgewählt aus der Gruppe bestehend aus Kollagen und Proteoglykan, im Vergleich zur Bestimmung der Menge an synthetisierten extrazellulären Matrixproteinen, insbesondere ausgewählt aus der Gruppe bestehend aus Kollagen, Proteoglykan und Elastin, besonders bevorzugt ausgewählt aus der Gruppe bestehend aus Kollagen und Proteoglykan, in geeigneten Kontrollen mittels photometrischer Bestimmung der Menge an mit Farbstoff markierten synthetisierten extrazellulären Matrixproteinen, insbesondere ausgewählt aus der Gruppe bestehend aus Kollagen, Proteoglykan und Elastin, besonders bevorzugt ausgewählt aus der Gruppe bestehend aus Kollagen und Proteoglykan, bestimmt werden, insbesondere durch die in Beispiel 6 aufgeführten in vitro-Tests.

Erfindungsgemäß wird unter der Bezeichnung "biologische Wirksamkeit in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten" verstanden, dass die biologische Wirksamkeit der erfindungsgemäßen Kollagenpeptide mit einem der in den Beispielen 3 bis 6 explizit ausgeführten Assays gezeigt werden kann. Dabei können die einzelnen in den Beispielen 3 bis 6 aufgeführten Verfahrensparameter gegebenenfalls entsprechend dem fachmännischen Verständnis variiert werden, ohne die grundlegende Aussagekraft der Versuchsergebnisse zu beeinflussen. In bevorzugter Ausführungsform der vorliegenden Erfindung zeigen die erfindungsgemäßen Kollagenpeptide eine biologische Wirksamkeit in mindestens einem, bevorzugt in mindestens zwei, bevorzugt in allen, der in den Beispielen 3 bis 6 dargestellten in vitro-Tests zur Stimulation der Synthese von extrazellulären Matrixproteinen in Osteoblasten, Fibroblasten und Chondrocyten unter exakt den in diesen Beispielen aufgeführten Verfahrensparametern. Ferner kann die biologische Wirksamkeit der erfindungsgemäßen Kollagenpeptide auch mit weiteren dem Fachmann bekannten Tests, insbesondere in vitro-Tests, bevorzugt in vitro-Tests zur Stimulation der Synthese von extrazellulären Matrixproteinen in Osteoblasten, Fibroblasten und Chondrocyten, nachgewiesen werden.

Unter der Bezeichnung "Stimulation der Synthese von extrazellulären Matrixproteinen" wird erfindungsgemäß eine Anregung der Biosynthese von mindestens einem Protein der extrazellulären Matrix in Zellen, bevorzugt in Osteoblasten, Fibroblasten und/oder Chondrocyten, und/oder eine Anregung der Biosynthese von mindestens einer ein Protein der extrazellulären Matrix codierenden mRNA in Zellen, bevorzugt in Osteoblasten, Fibroblasten und/oder Chondrocyten, durch exogene Einflüsse, insbesondere durch die erfindungsgemäßen Kollagenpeptide, verstanden. Insbesondere wird unter der Bezeichnung "Stimulation der Synthese von extrazellulären Matrixproteinen" erfindungsgemäß eine Steigerung der von Zellen, bevorzugt Osteoblasten, Fibroblasten und/oder Chondrocyten, sezernierten Menge mindestens eines Protein der extrazellulären Matrix und/oder eine Steigerung der in Zellen, bevorzugt in Osteoblasten, Fibroblasten und/oder Chondrocyten, synthetisierten Menge mindestens einer ein Protein der extrazellulären Matrix codierenden mRNA durch exogene Einflüsse, insbesondere durch die erfindungsgemäßen Kollagenpeptide, verstanden.

Im Zusammenhang mit der vorliegenden Erfindung wird der Begriff "Kollagen" in fachüblicher Weise verstanden, insbesondere so wie beispielsweise in der WO 01/34646 definiert. In bevorzugter Ausführungsform betrifft der Begriff "Kollagen" die Kollagen-Typen I bis XXVII. In weiterer bevorzugter Ausführungsform wird unter dem Begriff "Kollagen" ein die Sequenz Glycin-Prolin, Glycin-4-Hydroxyprolin oder Glycin-X-4-Hydroxyprolin, bevorzugt das repetitive Motiv (Gly-X-Y)ₙ, aufweisendes Peptid verstanden, wobei X und Y jede Aminosäure sein können, vorzugsweise Prolin und 4-Hydroxyprolin sind. Besonders bevorzugt wird unter dem Begriff "Kollagen" ein das repetitive Motiv (Gly-Pro-Y)ₙ und/oder (Gly-X-Hyp)ₘ aufweisendes Peptid verstanden, wobei X und Y jede Aminosäure sein können.

Der Begriff "Gelatine" wird im Zusammenhang mit der vorliegenden Erfindung in fachüblicher Weise verstanden, insbesondere so wie beispielsweise in der WO 01/34646 definiert.

Im Zusammenhang mit der vorliegenden Erfindung wird unter dem Begriff "Kollagenpeptid" ein Peptid verstanden, das eine in Kollagen gemäß vorstehender Definition vorkommende Aminosäuresequenz aufweist, wobei es sich bei dem Peptid mindestens um ein Dipeptid, bevorzugt um ein Oligopeptid oder Polypeptid, handelt. Dabei kann das Kollagenpeptid insbesondere in chemisch-modifizierter Form, insbesondere hydroxylierter und/oder glykosylierter Form, vorliegen oder nicht modifiziert sein.

Der Begriff "Aminosäure-Modifikation" bezeichnet im Zusammenhang mit der vorliegenden Offenbarung eine vor, nach oder während der Synthese des Kollagenpeptids erfolgte chemische Veränderung einer oder mehrerer Aminosäuren unter Beibehaltung des ursprünglichen Aminosäuregrundgerüsts, insbesondere einer oder mehrerer proteinogener Aminosäuren, des Kollagenpeptids. Somit umfasst der Begriff sowohl den Einsatz von chemisch veränderten Aminosäuren zur Synthese des erfindungsgemäßen Kollagenpeptids als auch die chemische Veränderung der Aminosäuren nach oder während der Synthese des Kollagenpeptids. Für Kollagenpeptide typische Aminosäure-Modifikationen sind insbesondere Hydroxylierungen an Prolin- und Lysinresten sowie Glykosylierungen von hydroxylierten Lysinresten. Der Begriff umfasst erfindungsgemäß jedoch auch andere chemische Veränderungen von Aminosäuren, wie Phosphorylierungen, N-Glykosylierungen, Acetylierungen, Methylierungen, Myristoylierungen.

Im Zusammenhang mit der vorliegenden Erfindung wird unter einem "synthetischen oder rekombinanten Kollagenpeptid" beziehungsweise unter einem "synthetisch oder rekombinant hergestellten Kollagenpeptid" ein durch chemische Synthese, insbesondere Festphasensynthese, oder durch biotechnologische rekombinante Herstellung mittels eines Expressionssystems gewonnenes Kollagenpeptid verstanden. Erfindungsgemäß ist dem "synthetischen Kollagenpeptid" beziehungsweise dem "synthetisch hergestellten Kollagenpeptid" und dem "rekombinanten Kollagenpeptid" beziehungsweise dem "rekombinant hergestellten Kollagenpeptid" gemein, dass diese nicht aus natürlichen Quellen gewonnen werden.

Im Zusammenhang mit der vorliegenden Erfindung wird unter dem Begriff "rekombinante DNA" ein artifiziell hergestelltes oder manipuliertes DNA-Molekül bezeichnet, das in vitro mittels gentechnischer Methoden hergestellt wurde. In bevorzugter Ausführungsform setzt sich die rekombinante DNA aus Bestandteilen unterschiedlicher Herkunftsorganismen zusammen.

Im Zusammenhang mit der vorliegenden Erfindung wird unter dem Begriff "Expressionskassette" ein DNA-Segment verstanden, das für die Transkiption der in diesem Segment codierten Information in eine RNA, insbesondere in eine mRNA, verantwortlich ist und zumindest einen Promotor und eine proteincodierende Nukleotidsequenz, in der Regel mindestens einen Promotor, mindestens eine proteincodierende Nukleotidsequenz und gegebenenfalls einen Terminator aufweist.

Im Zusammenhang mit der vorliegenden Erfindung wird unter einer "Nukleotidsequenz" die Abfolge der Nukleotide einer Nukleinsäure, insbesondere eines Nukleinsäurestrangs, insbesondere eines DNA- oder RNA-Strangs, verstanden. Eine "Nukleotidsequenz" ist daher sowohl als informatorische Einheit als auch als der diese Information physisch manifestierende DNA- oder RNA-Strang zu verstehen.

Im Zusammenhang mit der vorliegenden Erfindung wird unter einem "Expressionssystem" ein System verstanden, in dem eine gezielte und kontrollierte Proteinbiosynthese erfolgen kann. Dabei umfasst der Begriff "Expressionssystem" erfindungsgemäß sowohl zellfreie Expressionssysteme, bei denen die zur Proteinbiosynthese notwendigen Komponenten nicht innerhalb einer Zelle vorliegen, das heißt die Proteinbiosynthese außerhalb einer Zelle erfolgt, als auch zellbasierte Expressionssysteme, bei denen die Proteinbiosynthese innerhalb einer lebenden Zelle erfolgt. Bei einem zellfreien Expressionssystem handelt es sich im Zusammenhang mit der vorliegenden Erfindung bevorzugt um ein Lysat oder ein Extrakt aus *E. coli,* Insektenzellen, Weizenkeimen, Tabakzellen oder Säugetierzellen, insbesondere CHO-Zellen oder Retikulocyten aus Kaninchen, welches die zur Proteinbiosynthese notwendigen Komponenten, insbesondere ein Translations- und ein Transkriptionssystem, aufweist. Im Falle der Verwendung eines zellfreien Expressionsystems in einem der Verfahren gemäß der vorliegenden Erfindung ist der Begriff "Kultivieren" gleichbedeutend mit "Inkubieren".

Im Zusammenhang mit der vorliegenden Erfindung wird unter einer "Wirtszelle" eine lebende Zelle verstanden, die zur Expression von in Fremd-DNA, insbesondere in rekombinanter DNA, codierten Peptiden oder Proteinen, befähigt ist.

Unter den Begriffen "Gewinnen des Kollagenpeptids" gemäß Verfahrensschritt c) wird ein dem Fachmann bekanntes Verfahren zur Isolierung des Kollagenpeptids aus einer mehrere Komponenten enthaltenden Zusammensetzung mittels bekannter Isolierungsverfahren, wie beispielsweise Zentrifugationsverfahren, insbesondere differenzielle Zentrifugation und/oder Dichtegradientenzentrifugation, chromatografischer Verfahren, insbesondere Gelfiltrations-, Ionenaustausch-, Affinitäts- und/oder Hochleistungsflüssigkeitschromatografie, Elektrophoreseverfahren, Filtrationsverfahren und/oder Extraktionsverfahren, verstanden, wobei eine Anreicherung und Reinigung der betreffenden Komponente aus der mehrere Komponenten enthaltenden Zusammensetzung bevorzugt durch sequenzielle Anwendung mehrerer Isolationsverfahren erzielt werden kann.

Unter "Bedingungen, die die Expression des Kollagenpeptids ermöglichen" werden Bedingungen, wie insbesondere Temperatur, Druck, Zeit, Licht sowie An- oder Abwesenheit von Induktoren und/oder Repressoren, verstanden, die eine Expression des Kollagenpeptids aktivieren oder verstärken. In einer bevorzugten Ausführungsform erfolgt die Expression des Kollagenpeptids in Rahmen einer Hochzelldichtefermentation, insbesondere unter Hochdruck, bevorzugt Luft-Hochdruck. Die konkreten Bedingungen, die eine Expression des Kollagenpeptids ermöglichen, sind dem Fachmann bekannt und hängen von dem verwendeten Expressionssystem und der verwendeten Expressionskassette, insbesondere des darin enthaltenen Promotors, ab. Bei der Expression des Kollagenpeptids kann es sich je nach Aufbau der Expressionskassette um eine konstitutive oder induzierbare Expression handeln.

Im Zusammenhang mit der vorliegenden Erfindung wird unter den Begriffen "umfassend" und "aufweisend" verstanden, dass zusätzlich zu den von diesen Begriffen explizit erfassten Elementen noch weitere, nicht explizit genannte Elemente hinzutreten können. Im Zusammenhang mit der vorliegenden Erfindung wird unter diesen Begriffen auch verstanden, dass allein die explizit genannten Elemente erfasst werden und keine weiteren Elemente vorliegen. In dieser besonderen Ausführungsform ist die Bedeutung der Begriffe "umfassend" und "aufweisend" gleichbedeutend mit dem Begriff "bestehend aus". Darüber hinaus erfassen die Begriffe "umfassend" und "aufweisend" auch Zusammensetzungen, die neben den explizit genannten Elementen auch weitere nicht genannte Elemente enthalten, die jedoch von funktionell und qualitativ untergeordneter Natur sind. In dieser Ausführungsform sind die Begriffe "umfassend" und "aufweisend" gleichbedeutend mit dem Begriff "im Wesentlichen bestehend aus".

Sofern im Zusammenhang mit der vorliegenden Erfindung die erste und zweite Nachkommastelle oder die zweite Nachkommastelle nicht angegeben sind/ist, sind/ist diese als 0 zu setzen.

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: a) A oder B oder C oder b) (A und B) oder c) (A und C) oder d) (B und C) oder e) (A und B und C).

Weitere bevorzugte Ausführungsformen ergeben sich aus den Unteransprüchen.

Die Erfindung wird nachstehend ohne Beschränkung des allgemeinen Erfindungsgedankens anhand beispielhafter Sequenzen, Figuren und Ausführungsbeispielen beschrieben.

Dabei bezeichnet:
**SEQ ID No. 1** die Aminosäuresequenz **Leu-Thr-Gly-Pro-Ile-Gly-Pro-Pro-Gly-Pro-Ala** eines 11 Aminosäuren umfassenden nicht-hydroxylierten Kollagenpeptids.
**SEQ ID No. 2** die Aminosäuresequenz **Gly-Ala-Pro-Gly-Lys-Asp-Gly-Val-Arg-Gly-Leu-Thr-Gly-Pro-Ile-Gly-Pro-Pro-Gly-Pro-Ala-Gly-Ala-Pro-Gly-Asp-Lys-Gly-Glu-Ala-Gly-Pro-Ser** eines 33 Aminosäuren umfassenden nicht-hydroxylierten Kollagenpeptids.
**SEQ ID No. 3** die Aminosäuresequenz **Gly-Ala-Pro-Gly-Lys-Asp-Gly-Val-Arg-Gly-Leu-Thr-Gly-Pro-Ile-Gly-Pro-Pro-Gly-Pro-Ala-Gly-Ala-Pro** eines 24 Aminosäuren umfassenden nicht-hydroxylierten Kollagenpeptids.
**SEQ ID No. 4** die Aminosäuresequenz **Gly-Ala-Pro-Gly-Lys-Asp-Gly-Val-Arg-Gly-Leu-Thr-Gly-Pro-Ile-Gly-Pro-Pro-Gly-Pro-Ala-Gly-Ala-Arg** eines 24 Aminosäuren umfassenden nicht-hydroxylierten Kollagenpeptids.
**SEQ ID No. 5** die Aminosäuresequenz **Gly-Ala-Pro-Gly-Pro-Pro-Gly-Pro-Pro-Gly-Ala-Arg-Gly-Gln-Ala-Gly-Val-Met-Gly-Phe-Pro-Gly-Pro-Lys** eines 24 Aminosäuren umfassenden nicht-hydroxylierten Kollagenpeptids.
**SEQ ID No. 6** die Aminosäuresequenz **Leu-Thr-Gly-Pro-Ile-Gly-Pro-4Hyp-Gly-Pro-Ala** eines 11 Aminosäuren umfassenden hydroxylierten Kollagenpeptids.
**SEQ ID No. 7** die Aminosäuresequenz **Gly-Ala-4Hyp-Gly-Lys-Asp-Gly-Val-Arg-Gly-Leu-Thr-Gly-Pro-Ile-Gly-Pro-4Hyp-Gly-Pro-Ala-Gly-Ala-4Hyp-Gly-Asp-Lys-Gly-Glu-Ala-Gly-Pro-Ser** eines 33 Aminosäuren umfassenden hydroxylierten Kollagenpeptids.
**SEQ ID No. 8** die Aminosäuresequenz **Gly-Ala-4Hyp-Gly-Lys-Asp-Gly-Val-Arg-Gly-Leu-Thr-Gly-Pro-Ile-Gly-Pro-4Hyp-Gly-Pro-Ala-Gly-Ala-4Hyp** eines 24 Aminosäuren umfassenden hydroxylierten Kollagenpeptids.
**SEQ ID No. 9** die Aminosäuresequenz **Gly-Ala-4Hyp-Gly-Lys-Asp-Gly-Val-Arg-Gly-Leu-Thr-Gly-Pro-Ile-Gly-Pro-4Hyp-Gly-Pro-Ala-Gly-Ala-Arg** eines 24 Aminosäuren umfassenden hydroxylierten Kollagenpeptids.
**SEQ ID No. 10** die Aminosäuresequenz **Gly-Ala-4Hyp-Gly-Pro-Pro-Gly-Pro-4Hyp-Gly-Ala-Arg-Gly-Gln-Ala-Gly-Val-Met-Gly-Phe-4Hyp-Gly-Pro-Lys** eines 24 Aminosäuren umfassenden hydroxylierten Kollagenpeptids.
**SEQ ID No. 11** die Aminosäuresequenz **Gly-Ala-Pro-Gly-Lys-Asp-Gly-Val-Arg-Gly-Leu** eines 11 Aminosäuren umfassenden nicht-hydroxylierten Kollagenpeptids.
**SEQ ID No. 12** die Aminosäuresequenz **Gly-Ala-Pro-Gly-Lys-Asp-Gly-Val-Arg-Gly-Leu-Thr-Gly-Pro-Ile-Gly-Pro-Pro-Gly-Pro-Ala-Gly-Ala** eines 23 Aminosäuren umfassenden nicht-hydroxylierten Kollagenpeptids.
**SEQ ID No. 13** die Aminosäuresequenz **Gly-Ala-Pro-Gly-Lys-Asp-Gly-Val-Arg-Gly-Leu-Thr-Gly-Pro-Ile-Gly-Pro-Pro-Gly-Pro-Ala-Gly-Ala-Pro-Gly-Asp-Lys-Gly-Glu-Ala-Gly-Pro-Ser-Gly-Pro-Ala-Gly-Pro-Thr-Gly-Ala-Arg-Gly-Ala-Pro-Gly-Asp-Arg-Gly-Glu-Pro-Gly-Pro-Pro-Gly-Pro-Ala-Gly** eines 58 Aminosäuren umfassenden nicht-hydroxylierten Kollagenpeptids.
**SEQ ID No. 14** die Aminosäuresequenz **Gly-Ala-Pro-Gly-Lys-Asp-Gly-Val-Arg-Gly-Leu-Thr-Gly-Pro-Ile-Gly-Pro-Pro-Gly-Pro-Ala-Gly-Ala-Pro-Gly-Asp-Lys-Gly-Glu-Ala-Gly-Pro-Ser-Gly-Pro-Ala-Gly-Pro-Thr-Gly-Ala-Arg-Gly-Ala-Pro-Gly-Asp-Arg-Gly-Glu-Pro-Gly-Pro-Pro-Gly-Pro-Ala-Gly-Phe-Ala-Gly-Pro-Pro-Gly-Ala-Asp-Gly-Gln-Pro-Gly-Ala-Lys-Gly-Glu-Pro-Gly-Asp-Ala-Gly-Ala-Lys-Gly-Asp-Ala-Gly-Pro-Pro-Gly-Pro-Ala** eines 90 Aminosäuren umfassenden nicht-hydroxylierten Kollagenpeptids.
**SEQ ID No. 15** die Aminosäuresequenz **Lys-Gly-Ala-Pro-Gly-Ala-Asp-Gly-Pro-Ala-Gly-Ala-Pro-Gly-Thr-Pro-Gly-Pro-Gln** eines 19 Aminosäuren umfassenden nicht-hydroxylierten Kollagenpeptids.
**SEQ ID No. 16** die Aminosäuresequenz **Gly-Pro-Pro-Gly-Pro-Ala-Gly-Glu-Lys-Gly-Ala-Pro-Gly-Ala-Asp-Gly-Pro-Ala-Gly-Ala-Pro-Gly-Thr-Pro-Gly-Pro-Gln-Gly-Ile-Ala-Gly-Gln-Arg-Gly-Val** eines 35 Aminosäuren umfassenden nicht-hydroxylierten Kollagenpeptids.
**SEQ ID No. 17** die Aminosäuresequenz **Gly-Ala-4Hyp-Gly-Lys-Asp-Gly-Val-Arg-Gly-Leu** eines 11 Aminosäuren umfassenden hydroxylierten Kollagenpeptids.
**SEQ ID No. 18** die Aminosäuresequenz **Gly-Ala-4Hyp-Gly-Lys-Asp-Gly-Val-Arg-Gly-Leu-Thr-Gly-Pro-Ile-Gly-Pro-4Hyp-Gly-Pro-Ala-Gly-Ala** eines 23 Aminosäuren umfassenden hydroxylierten Kollagenpeptids.
**SEQ ID No. 19** die Aminosäuresequenz **Gly-Ala-4Hyp-Gly-Lys-Asp-Gly-Val-Arg-Gly-Leu-Thr-Gly-Pro-Ile-Gly-Pro-4Hyp-Gly-Pro-Ala-Gly-Ala-4Hyp-Gly-Asp-Lys-Gly-Glu-Ala-Gly-Pro-Ser-Gly-Pro-Ala-Gly-Pro-Thr-Gly-Ala-Arg-Gly-Ala-4Hyp-Gly-Asp-Arg-Gly-Glu-4Hyp-Gly-Pro-4Hyp-Gly-Pro-Ala-Gly** eines 58 Aminosäuren umfassenden hydroxylierten Kollagenpeptids.
**SEQ ID No. 20** die Aminosäuresequenz **Gly-Ala-4Hyp-Gly-Lys-Asp-Gly-Val-Arg-Gly-Leu-Thr-Gly-Pro-Ile-Gly-Pro-4Hyp-Gly-Pro-Ala-Gly-Ala-4Hyp-Gly-Asp-Lys-Gly-Glu-Ala-Gly-Pro-Ser-Gly-Pro-Ala-Gly-Pro-Thr-Gly-Ala-Arg-Gly-Ala-4Hyp-Gly-Asp-Arg-Gly-Glu-4Hyp-Gly-Pro-4Hyp-Gly-Pro-Ala-Gly-Phe-Ala-Gly-Pro-4Hyp-Gly-Ala-Asp-Gly-Gln-4Hyp-Gly-Ala-Lys-Gly-Glu-Pro-Gly-Asp-Ala-Gly-Ala-Lys-Gly-Asp-Ala-Gly-Pro-Pro-Gly-Pro-Ala** eines 90 Aminosäuren umfassenden hydroxylierten Kollagenpeptids.
**SEQ ID No. 21** die Aminosäuresequenz **Lys-Gly-Ala-4Hyp-Gly-Ala-Asp-Gly-Pro-Ala-Gly-Ala-4Hyp-Gly-Thr-Pro-Gly-Pro-Gln** eines 19 Aminosäuren umfassenden hydroxylierten Kollagenpeptids.
**SEQ ID No. 22** die Aminosäuresequenz **Gly-Pro-4Hyp-Gly-Pro-Ala-Gly-Glu-Lys-Gly-Ala-4Hyp-Gly-Ala-Asp-Gly-Pro-Ala-Gly-Ala-4Hyp-Gly-Thr-Pro-Gly-Pro-Gln-Gly-Ile-Ala-Gly-Gln-Arg-Gly-Val** eines 35 Aminosäuren umfassenden hydroxylierten Kollagenpeptids.
**SEQ ID No. 23** die Aminosäuresequenz **Gly-Val-4Hyp-Gly-Lys-Tyr-Gly-Val-Arg-Gly-Leu-Thr-Gly-Pro-Ile-Gly-Pro-4Hyp-Gly-Pro-Leu-Gly-Ile-4Hyp-Gly-Trp-Lys-Gly-Phe-Val-Gly-Pro-Thr** eines 33 Aminosäuren umfassenden hydroxylierten Kollagenpeptids.
**SEQ ID No. 24** die Aminosäuresequenz **Gly-Pro-4Hyp-Gly-Pro-Val-Gly-Thr-Lys-Gly-Ile-4Hyp-Gly-Val-Tyr-Gly-Pro-Leu-Gly-Ile-4Hyp-Gly-Thr-Pro-Gly-Pro-Trp-Gly-Ile-Leu-Gly-Thr-Lys-Arg-Gly-Val** eines 36 Aminosäuren umfassenden hydroxylierten Kollagenpeptids.
**SEQ ID No. 25** die Aminosäuresequenz **Gly-Ala-4Hyp-Gly-Lys-Asp-Gly-Val-Arg-Gly-Leu-Thr-Gly-4Hyp-Ile-Gly-4Hyp-4Hyp-Gly-4Hyp-Ala-Gly-Ala-4Hyp-Gly-Asp-Lys-Gly-Glu-Ala-Gly-4Hyp-Ser** eines 33 Aminosäuren umfassenden hydroxylierten Kollagenpeptids.
**SEQ ID No. 26** die Aminosäuresequenz **His-His-His-His-His-His-Gly-Ala-4Hyp-Gly-Lys-Asp-Gly-Val-Arg-Gly-Leu-Thr-Gly-Pro-Ile-Gly-Pro-4Hyp-Gly-Pro-Ala-Gly-Ala-4Hyp-Gly-Asp-Lys-Gly-Glu-Ala-Gly-Pro-Ser** eines 39 Aminosäuren umfassenden hydroxylierten Kollagenpeptids.
**SEQ ID No. 27** die Aminosäuresequenz **Gly-Leu-4Hyp-Gly-Lys-Met-Gly-Val-Phe-Gly-Leu-Thr-Gly-Pro-Ile-Gly-Pro-4Hyp-Gly-Pro-Trp-Gly-Val-4Hyp-Gly-His-Lys-Gly-Tyr-Leu-Gly-Pro-Thr** eines 33 Aminosäuren umfassenden hydroxylierten Kollagenpeptids.
**SEQ ID No. 28** die Aminosäuresequenz **Gly-Leu-4Hyp-Gly-Lys-Tyr-Gly-Val-His-Gly-Leu-Thr-Gly-Pro-Leu-Gly-Pro-4Hyp-Gly-Pro-Met-Gly-Ile-4Hyp-Gly-Trp-Lys-Gly-Phe-Val-Gly-Pro-Thr** eines 33 Aminosäuren umfassenden hydroxylierten Kollagenpeptids.
**SEQ ID No. 29** die Aminosäuresequenz **Gly-Pro-4Hyp-Gly-Pro-Leu-Gly-Met-Lys-Gly-Leu-4Hyp-Gly-Val-Trp-Gly-Pro-Phe-Gly-Leu-4Hyp-Gly-Thr-Pro-Gly-Pro-His-Gly-Ile-Thr-Gly-Tyr-Lys-Gly-Val** eines 35 Aminosäuren umfassenden hydroxylierten Kollagenpeptids.
**SEQ ID No. 30** die Aminosäuresequenz **Gly-Pro-4Hyp-Gly-Pro-Val-Gly-Thr-Lys-Gly-Leu-4Hyp-Gly-Phe-Tyr-Gly-Pro-Leu-Gly-Ile-4Hyp-Gly-His-Pro-Gly-Pro-Trp-Gly-Met-Leu-Gly-Thr-Lys-Gly-Val** eines 35 Aminosäuren umfassenden hydroxylierten Kollagenpeptids.

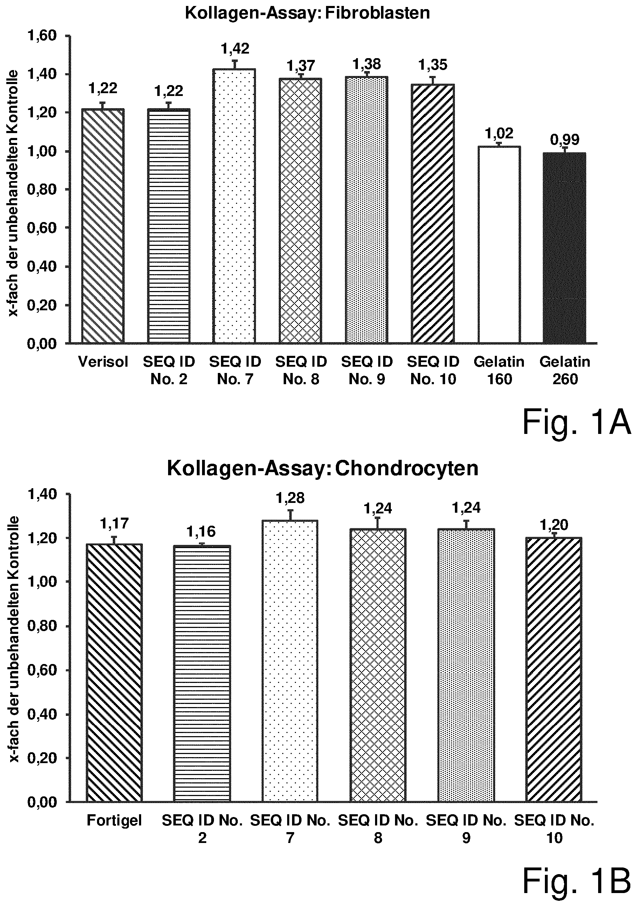

Es zeigt
**Figur 1A** die Stimulation der Kollagensynthese von humanen dermalen Fibroblasten durch die erfindungsgemäßen Kollagenpeptide der SEQ ID No. 2, SEQ ID No. 7, SEQ ID No. 8, SEQ ID No. 9, SEQ ID No. 10 im Vergleich zu einem Gemisch von aus natürlichen Quellen gewonnenen Kollagenpeptiden mit vergleichbarem durchschnittlichem Molekulargewicht (Verisol) und im Vergleich zu zwei unterschiedlichen Gelatine-Kontrollen (160 Bloom Gelatine aus Schweineschwarten (Gelatin 160), 260 Bloom Gelatine aus Rinderspalt (Gelatin 260)) gemäß Beispiel 6. Dargestellt ist der Faktor der quantitativen Bestimmung der Kollagensynthese gegenüber einer unbehandelten Probe. Die Fehlerbalken zeigen jeweils den Standardfehler (SEM).
**Figur 1B** die Stimulation der Kollagensynthese von humanen Chondrocyten durch die erfindungsgemäßen Kollagenpeptide der SEQ ID No. 2, SEQ ID No. 7, SEQ ID No. 8, SEQ ID No. 9, SEQ ID No. 10 im Vergleich zu einem Gemisch von aus natürlichen Quellen gewonnenen Kollagenpeptiden mit vergleichbarem durchschnittlichem Molekulargewicht (Fortigel) gemäß Beispiel 6. Dargestellt ist der Faktor der quantitativen Bestimmung der Kollagensynthese gegenüber einer unbehandelten Probe. Die Fehlerbalken zeigen jeweils den Standardfehler (SEM).

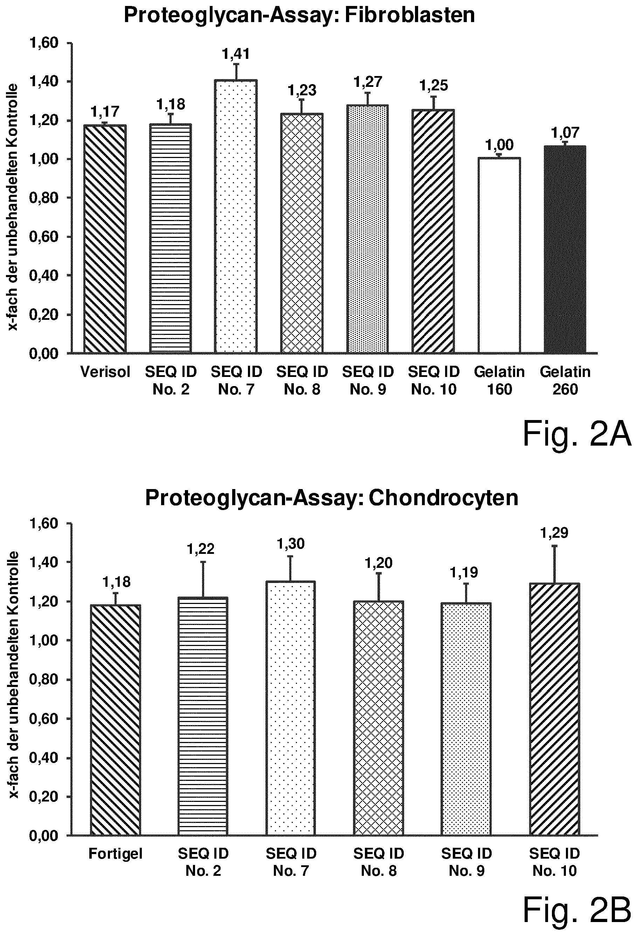

**Figur 2A** die Stimulation der Synthese von Proteoglykanen von humanen dermalen Fibroblasten durch die erfindungsgemäßen Kollagenpeptide der SEQ ID No. 2, SEQ ID No. 7, SEQ ID No. 8, SEQ ID No. 9, SEQ ID No. 10 im Vergleich zu einem Gemisch von aus natürlichen Quellen gewonnenen Kollagenpeptiden mit vergleichbarem durchschnittlichem Molekulargewicht (Verisol) und im Vergleich zu zwei unterschiedlichen Gelatine-Kontrollen (160 Bloom Gelatine aus Schweineschwarten (Gelatin 160), 260 Bloom Gelatine aus Rinderspalt (Gelatin 260)) gemäß Beispiel 6. Dargestellt ist der Faktor der quantitativen Bestimmung der Synthese von Proteoglykanen gegenüber einer unbehandelten Probe. Die Fehlerbalken zeigen jeweils den Standardfehler (SEM).
**Figur 2B** die Stimulation die Synthese von Proteoglykanen von humanen Chondrocyten durch die erfindungsgemäßen Kollagenpeptide der SEQ ID No. 2, SEQ ID No. 7, SEQ ID No. 8, SEQ ID No. 9, SEQ ID No. 10 im Vergleich zu einem Gemisch von aus natürlichen Quellen gewonnenen Kollagenpeptiden mit vergleichbarem durchschnittlichem Molekulargewicht (Fortigel) gemäß Beispiel 6. Dargestellt ist der Faktor der quantitativen Bestimmung der Kollagensynthese gegenüber einer unbehandelten Probe. Die Fehlerbalken zeigen jeweils den Standardfehler (SEM).

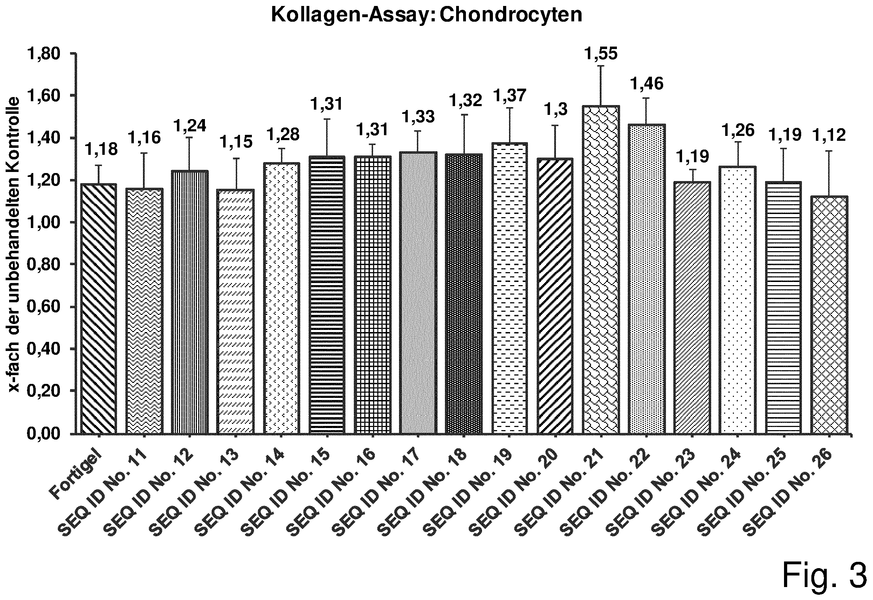

**Figur 3** die Stimulation der Kollagensynthese von humanen Chondrocyten durch die erfindungsgemäßen Kollagenpeptide der SEQ ID No. 11 bis SEQ ID No. 26 im Vergleich zu einem Gemisch von aus natürlichen Quellen gewonnenen Kollagenpeptiden mit vergleichbarem durchschnittlichem Molekulargewicht (Fortigel). Dargestellt ist der Faktor der quantitativen Bestimmung der Kollagensynthese gegenüber einer unbehandelten Probe. Die Fehlerbalken zeigen jeweils den Standardfehler (SEM).

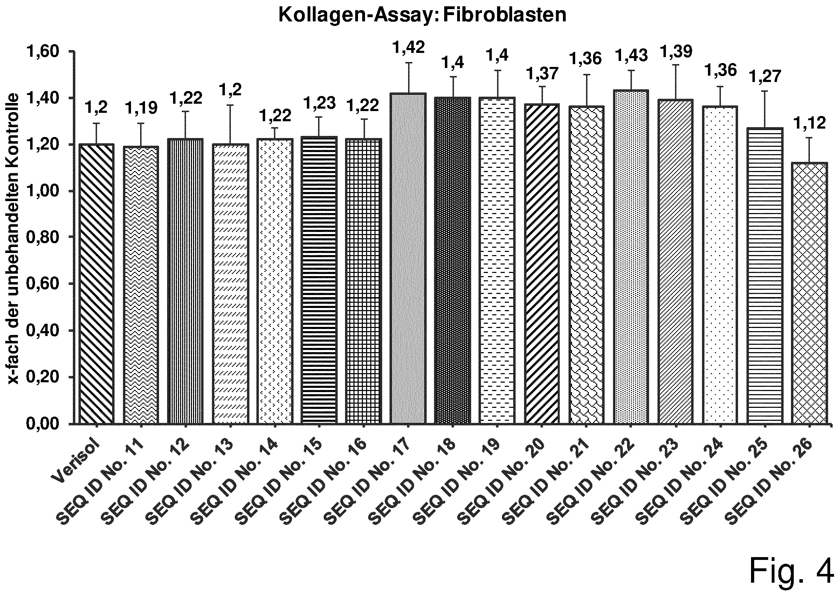

**Figur 4** die Stimulation der Kollagensynthese von humanen dermalen Fibroblasten durch die erfindungsgemäßen Kollagenpeptide der SEQ ID No. 11 bis SEQ ID No. 26 im Vergleich zu einem Gemisch von aus natürlichen Quellen gewonnenen Kollagenpeptiden mit vergleichbarem durchschnittlichem Molekulargewicht (Verisol). Dargestellt ist der Faktor der quantitativen Bestimmung der Kollagensynthese gegenüber einer unbehandelten Probe. Die Fehlerbalken zeigen jeweils den Standardfehler (SEM).

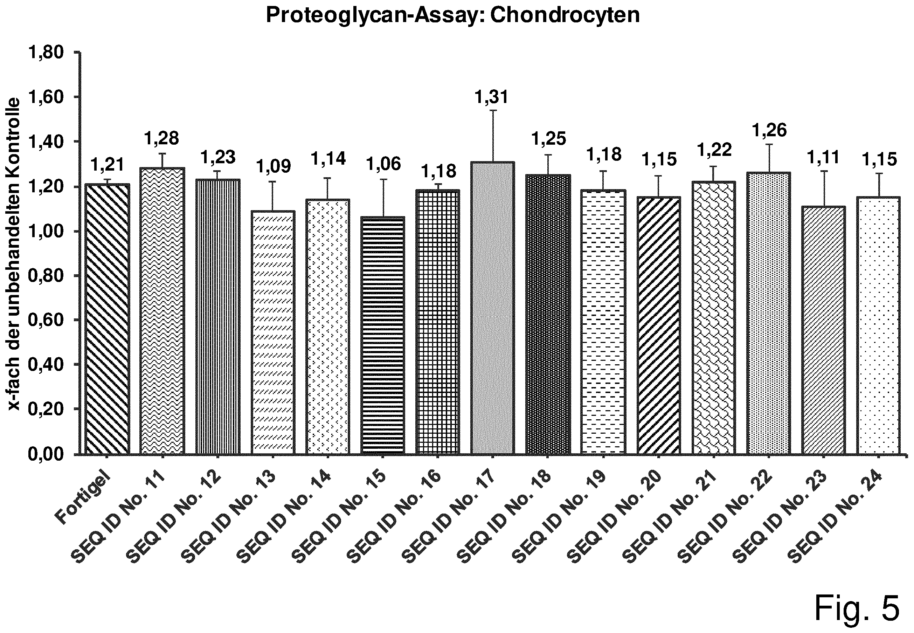

**Figur 5** die Stimulation der Synthese von Proteoglykanen von humanen Chondrocyten durch die erfindungsgemäßen Kollagenpeptide der SEQ ID No. 11 bis SEQ ID No. 24 im Vergleich zu einem Gemisch von aus natürlichen Quellen gewonnenen Kollagenpeptiden mit vergleichbarem durchschnittlichem Molekulargewicht (Fortigel). Dargestellt ist der Faktor der quantitativen Bestimmung der Kollagensynthese gegenüber einer unbehandelten Probe. Die Fehlerbalken zeigen jeweils den Standardfehler (SEM).

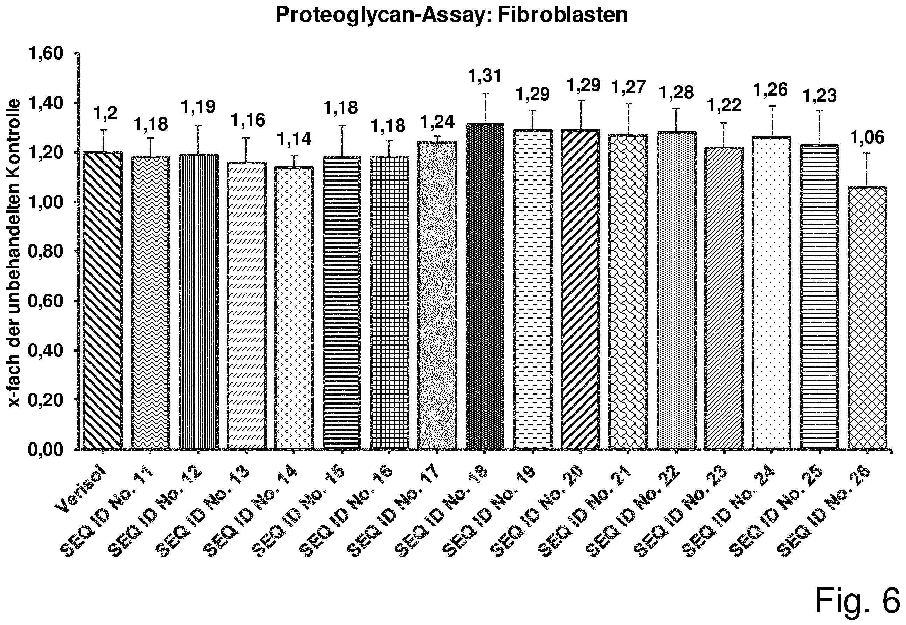

**Figur 6** die Stimulation der Synthese von Proteoglykanen von humanen dermalen Fibroblasten durch die erfindungsgemäßen Kollagenpeptide der SEQ ID No. 11 bis SEQ ID No. 26 im Vergleich zu einem Gemisch von aus natürlichen Quellen gewonnenen Kollagenpeptiden mit vergleichbarem durchschnittlichem Molekulargewicht (Verisol). Dargestellt ist der Faktor der quantitativen Bestimmung der Kollagensynthese gegenüber einer unbehandelten Probe. Die Fehlerbalken zeigen jeweils den Standardfehler (SEM).

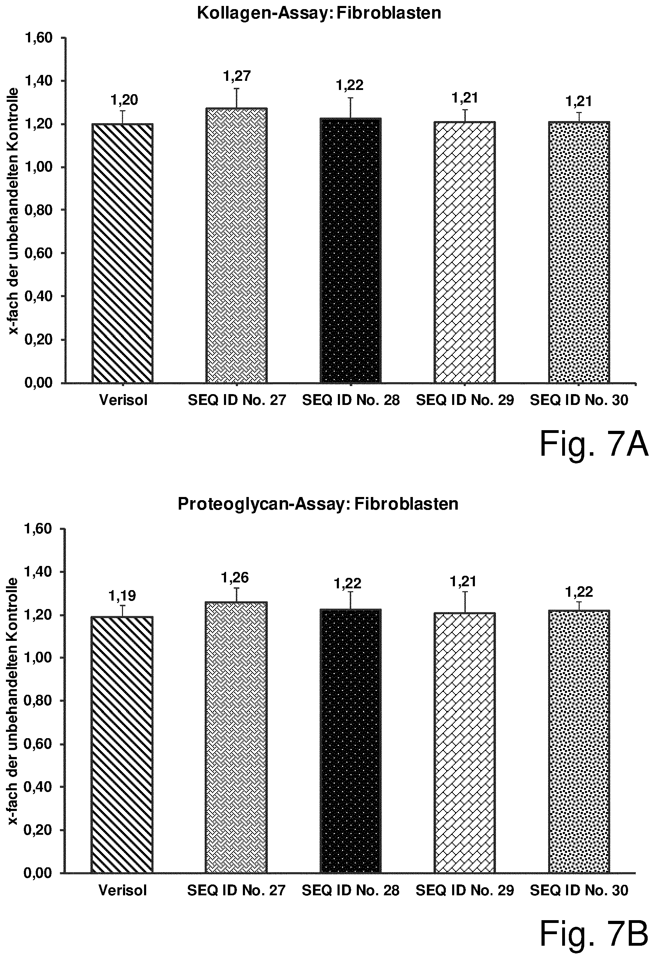

**Figur 7A** die Stimulation der Kollagensynthese von humanen dermalen Fibroblasten durch die erfindungsgemäßen Kollagenpeptide der SEQ ID No. 27 bis SEQ ID No. 30 im Vergleich zu einem Gemisch von aus natürlichen Quellen gewonnenen Kollagenpeptiden mit vergleichbarem durchschnittlichem Molekulargewicht (Verisol). Dargestellt ist der Faktor der quantitativen Bestimmung der Kollagensynthese gegenüber einer unbehandelten Probe. Die Fehlerbalken zeigen jeweils den Standardfehler (SEM).
**Figur 7B** die Stimulation der Synthese von Proteoglykanen von humanen dermalen Fibroblasten durch die erfindungsgemäßen Kollagenpeptide der SEQ ID No. 27 bis SEQ ID No. 30 im Vergleich zu einem Gemisch von aus natürlichen Quellen gewonnenen Kollagenpeptiden mit vergleichbarem durchschnittlichem Molekulargewicht (Verisol). Dargestellt ist der Faktor der quantitativen Bestimmung der Kollagensynthese gegenüber einer unbehandelten Probe. Die Fehlerbalken zeigen jeweils den Standardfehler (SEM).

### Beispiele:

### Beispiel 1 - Festphasensynthese

Erfindungsgemäße Kollagenpeptide der Aminosäuresequenzen SEQ ID No. 1 bis SEQ ID No. 10 und SEQ ID No. 27 bis SEQ ID No. 30 wurden durch Festphasensynthese (Merrifield-Synthese) an einem Polystyrol-Harz gewonnen.

### Beispiel 2 - rekombinante Herstellung

Weitere Kollagenpeptide, insbesondere die Kollagenpeptide der Aminosäuresequenzen SEQ ID No. 1 bis SEQ ID No. 10, wurden zusätzlich durch rekombinante Expression in *Pichia pastoris* gewonnen.

### Beispiel 3 - Knochengesundheit:

Zur Analyse der biologischen Wirksamkeit des erfindungsgemäßen Kollagenpeptids in Hinblick auf den Erhalt der Knochengesundheit und die Prophylaxe und Behandlung von Knochenerkrankungen wird dessen stimulierende Wirkung auf die Synthese von extrazellulären Matrixproteinen und Enzymen, die beim Aufbau und der Mineralisation der Matrix eine Rolle spielen, durch Osteoblasten in vitro untersucht. Dies erfolgt durch eine Bestimmung der Expression der entsprechenden mRNA mittels Realtime-PCR und eine semiquantitative Auswertung (bezogen auf eine Kontrolle ohne Kollagenpeptid).

Dazu werden zunächst humane Osteoblasten aus Kniegelenken isoliert, indem Knochenmaterial unter starker Agitation bei 37 °C für 1 h in Hanks-Lösung inkubiert wird, supplementiert mit 7 mg/ml Hyaluronidase Typ I und III-S und 5 mg/ml Pronase. Der Aufschluss wird anschließend bei 37 °C für 3-5 h in Hanks-Lösung, supplementiert mit 16 mg/ml Kollagenase Typ CLS IV, fortgesetzt. Die erhaltenen primäre Osteoblasten werden nach dem enzymatischen Aufschluss in Ham's F12-Medium, supplementiert mit 10% fötalem Kälberserum, 20 U/ml Penicillin-Streptomycin, 50 µg/ml Partricin, 0,05 mg/ml Ascorbinsäure und 0,15 mg/ml Glutamin kultiviert. Alternativ können primäre Osteoblasten (Artikel-Nr. C-12760; 2019) zur Untersuchung der biologischen Wirksamkeit auch von der PromoCell GmbH, Heidelberg, Deutschland, bezogen werden. Die Kultivierung der Zellen erfolgt dann in Ham's F12-Medium, supplementiert mit 10% fötalem Kälberserum, 20 U/ml Penicillin-Streptomycin, 50 µg/ml Partricin und 0,15 mg/ml Glutamin.

Für die Untersuchung der biologischen Wirksamkeit werden Monolayer-Zellkulturen der isolierten humanen Osteoblasten für einen Zeitraum von 24 h in einem Medium inkubiert, welches mit 0,5 mg/ml des jeweiligen Kollagenpeptids supplementiert ist. Eine Kontrolle wird jeweils in Medium ohne Peptid inkubiert. Anschließend erfolgt die Bestimmung der jeweiligen mRNA-Expression.

### Beispiel 4 - Hautgesundheit:

Die Stimulation der Synthese von Kollagen (Typ I) sowie der Proteoglykane Biglykan und Versican wird in vitro an humanen dermalen Fibroblasten (Hautzellen) untersucht. Hierfür werden die Zellen für 24 Stunden mit jeweils 0,5 mg/ml eines niedermolekularen beziehungsweise des erfindungsgemäßen Kollagenpeptids inkubiert und anschließend die Expression von Kollagen-RNA, Biglykan-RNA und Versican-RNA mittels Realtime-PCR bestimmt und semiquantitativ (bezogen auf eine Kontrolle ohne Peptid) ausgewertet.

### Beispiel 5 - Knorpelgesundheit:

Für die Zellkulturen werden porcine bzw. humane Chondrocyten auf bekannte Weise aus Knorpelgewebe isoliert und mit einer Dichte von ca. 350.000 Zellen/cm² auf Kulturplatten ausgesät. Als Kulturmedium wird Ham's F12-Medium mit 10 % fötalem Kälberserum, 10 µg/ml Gentamycin und 5 µg/ml Amphotericin B verwendet. Alternativ zu 10 µg/ml Gentamycin können auch 10 µg/ml Penicillin-Streptomycin verwendet werden. Die Kultivierung erfolgte bei 37 °C in einer sauerstoffreduzierten Atmosphäre (5 % O₂, 5 % CO₂ und 90 % N₂).

Bestimmung der Kollagen-Biosynthese:
Die Quantifizierung des von den Chondrocyten synthetisierten Kollagens (im Wesentlichen Typ II) erfolgt durch radioaktive Markierung mit ¹⁴C-Prolin, welches in das Kollagen eingebaut wird.

Dem Kulturmedium wird zunächst radioaktives ¹⁴C-Prolin zugesetzt und die Chondrocyten werden unter diesen Bedingungen bis zum Zeitpunkt der Bestimmung kultiviert. Um bei der Detektion das eingebaute von nicht eingebautem ¹⁴C-Prolin unterscheiden zu können, wird das isotopenhaltige Kulturmedium dann für einen Zeitraum von 3 Tagen durch reines Kulturmedium ersetzt. Anschließend wird das Kulturmedium verworfen und der adhärente Zellrasen wird mit destilliertem Wasser versetzt, um durch osmotischen Stress die Zellmembranen zu zerstören und cytosolisches, nicht gebundenes ¹⁴C-Prolin freizusetzen. Durch Zentrifugation werden die Zelltrümmer mit der synthetisierten extrazellulären Matrix pelletiert. Das Pellet wird in frischem destillierten Wasser resuspendiert und mit einem Xylen-Scintillations-Cocktail versetzt. Durch Detektion des ¹⁴C-Prolins mit einem Betacounter kann dann die Menge des synthetisierten Kollagens quantifiziert werden.

Alternativ kann die Quantifizierung mit Hilfe des Sircol Collagen Assay Kits (Artikel-Nr. 054S5000, 2019, tebu-bio, Offenbach, Deutschland, beziehungsweise Biocolor Ltd., UK) nach Herstellerangaben durchgeführt werden (siehe Beispiel 6).

Bestimmung der Proteoglykan-Biosynthese:
Die Quantifizierung der von den Chondrocyten synthetisierten Proteoglykane erfolgt durch eine Alcianblau-Färbung und photometrische Bestimmung der Glykosaminoglykane (GAG), welche Bestandteile der Proteoglykane sind.

Um den Gehalt an GAG in der Zellkultur zu bestimmen, wird zunächst das Kulturmedium verworfen und der adhärente Zellrasen wird mit PBS-Puffer (pH 7) gespült. Anschließend werden die Zellen bei 4 °C für 2 Stunden in einer 10 %-igen Formaldehydlösung in PBS fixiert. Nach Entfernen des Formaldehyds wird das Alcianblau-Färbereagenz (5 % Alcianblau in 3 %-iger Essigsäure) auf den Zellrasen gegeben und über Nacht bei 4 °C inkubiert. Nicht gebundenes Alcianblau wird verworfen und durch drei- bis viermaliges vorsichtiges Spülen mit PBS ausgewaschen. Durch Zugabe von saurer Guanidinlösung (8 mol/l) werden die GAG-Komplexe aus dem Zellrasen herausgelöst. Die Menge an Glykosaminoglykanen kann dann photometrisch bei einer Wellenlänge von 620 nm quantifiziert werden.

Alternativ kann die Quantifizierung mit Hilfe des Blyscan Glycosaminoglycan Assay Kits (Artikel-Nr. 054B3000, 2019, tebu-bio, Offenbach, Deutschland, beziehungsweise Biocolor Ltd., UK) nach Herstellerangaben durchgeführt werden (siehe Beispiel 6).

### Beispiel 6 - Einfluss erfindungsgemäßer synthetischer Kollagenpeptide auf die Biosynthese von Matrixproteinen

### Zellkultur:

Die verwendeten humanen Zellen wurden von tebu-bio GmbH, Offenbach, Deutschland erhalten.

Die Chondrocyten (Kat.-Nr. 402-05a) bzw. dermalen Fibroblasten (Kat.-Nr. 106-05a) wurden zunächst in 12-well Kulturplatten ausgesät und bei 37 °C, 5% CO₂ in Ham's F12 Medium kultiviert, dem 10% fötales Kälberserum, 20 U/ml Penicillin-Streptomycin und 50 µg/ml Ascorbinsäure zugegeben wurden. An jedem zweiten Tag wurde das Kulturmedium durch neues Kulturmedium ersetzt bis eine Konfluenz der Zellen von 80% erreicht war. Zur Untersuchung des Einflusses der erfindungsgemäßen Kollagenpeptide der SEQ ID No. 2 (nicht-hydroxyliert), SEQ ID No. 7 (hydroxyliert), SEQ ID No. 8 (hydroxyliert), SEQ ID No. 9 (hydroxyliert), SEQ ID No. 10 (hydroxyliert), SEQ ID No. 11 (nicht-hydroxyliert), SEQ ID No. 12 (nicht-hydroxyliert), SEQ ID No. 13 (nicht-hydroxyliert), SEQ ID No. 14 (nicht-hydroxyliert), SEQ ID No. 15 (nicht-hydroxyliert), SEQ ID No. 16 (nicht-hydroxyliert), SEQ ID No. 17 (hydroxyliert), SEQ ID No. 18 (hydroxyliert), SEQ ID No. 19 (hydroxyliert), SEQ ID No. 20 (hydroxyliert), SEQ ID No. 21 (hydroxyliert), SEQ ID No. 22 (hydroxyliert), SEQ ID No. 23 (hydroxyliert), SEQ ID No. 24 (hydroxyliert), SEQ ID No. 25 (hydroxyliert), SEQ ID No. 26 (hydroxyliert), SEQ ID No. 27 (hydroxyliert), SEQ ID No. 28 (hydroxyliert), SEQ ID No. 29 (hydroxyliert) und SEQ ID No. 30 (hydroxyliert) auf die Biosynthese von Matrixproteinen wurde im Anschluss das Zellkulturmedium durch ein spezielles Stimulationsmedium, dem 0,5 mg/ml der spezifischen Kollagenpeptide zugegeben waren, ersetzt.

### Kollagen-Assay:

Zur Untersuchung des Kollagenmetabolismus wurde die Menge an neu synthetisiertem Kollagen nach dreiwöchiger Stimulation der Zellen mit 0,5 mg/ml BCP bestimmt. Das synthetisierte Kollagen wurde mittels des Sircol-Assays (Artikel-Nr. 054S5000, 2019, tebu-bio, Offenbach, Deutschland, beziehungsweise Biocolor Ltd., UK) gemäß den Angaben des Herstellers isoliert. Kurz gefasst wurde zunächst jeweils das Kulturmedium verworfen und die adhärente Zellschichten mit 0,1 mg Pepsin-Lösung in 0,5 M Essigsäure bei 4 °C über Nacht verdaut. Die Zellsuspensionen wurden durch die Zugabe von 100 µl einer Säure-neutralisierenden Reagenz neutralisiert. Das synthetisierte Kollagen wurde anschließend durch die Zugabe von 200 µl einer Isolierungs- und Konzentrations-Lösung unter kräftigem Schütteln bei 4 °C über Nacht abgetrennt. Nach Zentrifugation (12 000 rpm, 10 min) und Verwerfen des Überstandes wurde das isolierte Kollagen in 1 ml Sircol-Farbstoff-Lösung resuspendiert. Nach 30 Minuten Schütteln und einer weiteren Zentrifugation wurde das Kollagen-Pellet mit 750 µl kalter saurer Feststoff-Waschreagenz überschichtet. Nach erneuter Zentrifugation wurde der Überstand erneut verworfen und das angereicherte Kollagen in 250 µl alkalischer Lösung aufgenommen. Jeweils 200 µl der Proben-Lösungen wurden zur photometrischen Quantifizierung des synthetisierten Kollagens verwendet. Die Extinktion wurde bei einer Wellenlänge von 492 nm gemessen. Dabei wurde die Menge des jeweils synthetisierten Kollagens auf Grundlage von standardisierten Kollagenlösungen bestimmt.

Dabei konnte eine im Vergleich zu unbehandelten Kontrollen gesteigerte Kollagensynthese durch dermale Fibroblasten festgestellt werden, die mit den erfindungsgemäßen hydroxylierten Kollagenpeptiden der SEQ ID No. 7 (42% Steigerung), SEQ ID No. 8 (37% Steigerung), SEQ ID No. 9 (38% Steigerung), SEQ ID No. 10 (35% Steigerung), SEQ ID No. 17 (42% Steigerung), SEQ ID No. 18 (40% Steigerung), SEQ ID No. 19 (40% Steigerung), SEQ ID No. 20 (37% Steigerung), SEQ ID No. 21 (36% Steigerung), SEQ ID No. 22 (43% Steigerung), SEQ ID No. 23 (39% Steigerung), SEQ ID No. 24 (36% Steigerung), SEQ ID No. 25 (27% Steigerung), SEQ ID No. 26 (12% Steigerung), SEQ ID No. 27 (27% Steigerung), SEQ ID No. 28 (22% Steigerung), SEQ ID No. 29 (21% Steigerung) und SEQ ID No. 30 (21% Steigerung) inkubiert wurden (Fig. 1A; Fig. 4; Fig. 7A). Ferner zeigte sich, dass die Inkubation dermaler Fibroblasten mit erfindungsgemäßen nicht-hydroxylierten Kollagenpeptiden zumindest eine vergleichbare Stimulierung der Kollagensynthese bewirkt wie die Inkubation der Zellen mit einem Gemisch aus natürlichen Quellen gewonnener hydroxylierter Kollagenpeptide mit vergleichbarem durchschnittlichem Molekulargewicht (Verisol) (SEQ ID No. 2 (22% Steigerung), SEQ ID No. 11 (19 % Steigerung), SEQ ID No. 12 (22% Steigerung), SEQ ID No. 13 (20% Steigerung), SEQ ID No. 14 (22% Steigerung), SEQ ID No. 15 (23% Steigerung) und SEQ ID No. 16 (22% Steigerung)). Alle untersuchten erfindungsgemäßen Kollagenpeptide zeigten eine signifikante Steigerung der Kollagensynthese im Vergleich zu Gelatine-Kontrollen (Gelatin 160, Gelatin 260).

Auch für Chondrocyten, die mit den erfindungsgemäßen Kollagenpeptiden inkubiert wurden, konnte im Vergleich zu unbehandelten Kontrollen eine gesteigerte Kollagensynthese beobachtet werden. Dabei führte die Inkubation von Chondrocyten mit hydroxylierten Kollagenpeptiden zu einer Steigerung der Kollagensynthese um 28% (SEQ ID No. 7), 24% (SEQ ID Nos. 8, 9), 20% (SEQ ID No. 10), 33% (SEQ ID No. 17), 32% (SEQ ID No. 18), 37% (SEQ ID No. 19), 30% (SEQ ID No. 20), 55% (SEQ ID No. 21), 46% (SEQ ID No. 22), 19% (SEQ ID Nos. 23, 25), 26% (SEQ ID No. 24), und 12% (SEQ ID No. 26). Die Inkubation von Chondrocyten mit erfindungsgemäßen nicht-hydroxylierten Kollagenpeptiden führte zumindest zu einer vergleichbaren Stimulierung der Kollagensynthese wie die Inkubation von Zellen mit einem Gemisch aus natürlichen Quellen gewonnener hydroxylierter Kollagenpeptide mit vergleichbarem durchschnittlichem Molekulargewicht (Fortigel) (Fig 1B; Fig. 3). Dabei führte die Inkubation von Chondrocyten mit nicht-hydroxylierten Kollagenpeptiden zu einer Steigerung der Kollagensynthese um 16% (SEQ ID No. 2, 11), 24% (SEQ ID No. 12), 15% (SEQ ID No. 13), 28% (SEQ ID No. 14) und 31% (SEQ ID Nos. 15, 16).

### Proteoglykan-Assay:

Zur Bestimmung von Proteoglykanen wurde der Blyscan-Glycosaminoglycan-Assay (Artikel-Nr. 054B3000, 2019, tebu-bio, Offenbach, Deutschland, beziehungsweise Biocolor Ltd., UK) verwendet. Dabei wurde die Biosynthese von Proteoglykanen nach zweiwöchiger Stimulation dermaler Fibroblasten bestimmt. Gemäß den Angaben des Herstellers wurden die Zellschichten nach Verwerfen des Kulturmediums mit 1 ml Papain-Extraktions-Lösung überschichtet und für drei Stunden bei 65 °C unter kräftigem Schütteln inkubiert. Daraufhin wurden die Zellsuspensionen zentrifugiert (10 000 g, 10 min) und die Überstände wurden gesammelt. Nach der Zugabe von 1 ml Blyscan Farbstoff-Lösung und Schütteln (30 min) wurden die Überstände erneut zentrifugiert (12 000 rpm, 10 min) und anschließend verworfen. Die isolierten Proteoglykan-Pellets wurden in 500 µl Dissoziations-Lösung resuspendiert. Eine photometrische Bestimmung des synthetisierten Proteoglykans in jeweils 200 µl Probenlösung erfolgte bei einer Wellenlänge von 656 nm im Vergleich zu unbehandelten Kontrollexperimenten.

Wie aus Figs. 2A, 6 und 7B ersichtlich, war die Synthese von Proteoglykanen durch dermale Fibroblasten im Vergleich zu unbehandelten Kontrollen signifikant erhöht, wenn die Fibroblasten mit erfindungsgemäßen Kollagenpeptiden inkubiert wurden. So führte die Inkubation der Zellen mit hydroxylierten Kollagenpeptiden zu einer Steigerung der Synthese von Proteoglykanen um 41% (SEQ ID No. 7), 23% (SEQ ID No. 8, 25), 27% (SEQ ID No. 9, 21), 25% (SEQ ID No. 10), 24% (SEQ ID No. 17), 31% (SEQ ID No 18), 29% (SEQ ID Nos. 19, 20), 28% (SEQ ID No. 22), 22% (SEQ ID No. 23, 28, 30), 26% (SEQ ID No. 24, 27), 6% (SEQ ID No. 26) und 21% (SEQ ID No. 29). Es konnte zudem gezeigt werden, dass es bei Inkubation dermaler Fibroblasten mit erfindungsgemäßen nicht-hydroxylierten Kollagenpeptiden zumindest zu einer vergleichbaren Stimulierung der Synthese von Proteoglykanen kommt wie bei Inkubation der Zellen mit einem Gemisch aus natürlichen Quellen gewonnener hydroxylierter Kollagenpeptide mit vergleichbarem durchschnittlichem Molekulargewicht (Verisol). Dabei führte die Inkubation von dermalen Fibroblasten mit nicht-hydroxylierten Kollagenpeptiden zu einer Steigerung der Kollagensynthese um 18% (SEQ ID No. 2, 11, 15, 16), 19% (SEQ ID No. 12), 16% (SEQ ID No. 13) und 14% (SEQ ID No. 14). Für alle untersuchten erfindungsgemäßen Kollagenpeptide konnte eine signifikante Steigerung der Synthese von Proteoglykanen im Vergleich zu Gelatine-Kontrollen (Gelatin 160, Gelatin 260) beobachtet werden.

Auch Chondrocyten, die mit den erfindungsgemäßen Kollagenpeptiden inkubiert wurden, zeigen im Vergleich zu unbehandelten Kontrollen eine gesteigerte Synthese von Proteoglykanen (Fig. 2B und 5). Dabei führte die Inkubation von Chondrocyten mit hydroxylierten Kollagenpeptiden zu einer Steigerung der Kollagensynthese um 30% (SEQ ID No. 7), 20% (SEQ ID No. 8), 19% (SEQ ID No. 9), 29% (SEQ ID No. 10), 31% (SEQ ID No. 17), 25% (SEQ ID No. 18), 18% (SEQ ID No. 19), 15% (SEQ ID No. 20, 24), 22% (SEQ ID No. 21), 26% (SEQ ID No. 22, 24) und 11% (SEQ ID No. 23). Die Inkubation von Chondrocyten mit erfindungsgemäßen nicht-hydroxylierten Kollagenpeptiden führte zumindest zu einer vergleichbaren Stimulierung der Synthese von Proteoglykanen wie die Inkubation von Zellen mit einem Gemisch aus natürlichen Quellen gewonnenen hydroxylierten Kollagenpeptiden mit vergleichbarem durchschnittlichem Molekulargewicht (Fortigel) (Fig. 5; Fig. 2B). Dabei führte die Inkubation von Chondrocyten mit nicht-hydroxylierten Kollagenpeptiden zu einer Steigerung der Synthese von Proteoglykanen um 22% (SEQ ID No. 2), 28% (SEQ ID No. 11), 23% (SEQ ID No. 12), 9% (SEQ ID No. 13), 14% (SEQ ID No. 14), 6% (SEQ ID No.15) und 18% (SEQ ID No. 16).

## Patentansprüche

1. Ein synthetisches oder rekombinantes Kollagenpeptid zur Anwendung in einem Verfahren zur Prävention und/oder Behandlung von Knochenerkrankungen, Knorpelerkrankungen, degenerativen Gelenkerkrankungen, Erkrankungen der Sehnen oder Bänder und/oder Behandlung von Hauterkrankungen, wobei das Kollagenpeptid ein Molekulargewicht in einem Bereich von 0,18 bis 5,0 kDa aufweist und in der Lage ist, in Bindegewebszellen, insbesondere in Osteoblasten, Chondrocyten und/oder Fibroblasten, die Synthese von extrazellulären Matrixproteinen, insbesondere Kollagen, zu stimulieren und wobei das Kollagenpeptid eine Aminosäuresequenz ausgewählt aus der Gruppe bestehend aus SEQ ID No. 1 bis 30 aufweist.

2. Das Kollagenpeptid zur Anwendung nach Anspruch 1, wobei das Kollagenpeptid die Aminosäuresequenz SEQ ID No. 1 oder 15 aufweist.

3. Das Kollagenpeptid zur Anwendung nach Anspruch 1 oder 2, wobei das Kollagenpeptid die Aminosäuresequenz SEQ ID No. 1 aufweist, in der mindestens ein Prolinrest hydroxyliert ist, insbesondere wobei das Kollagenpeptid die Aminosäuresequenz SEQ ID No. 6 aufweist.

4. Das Kollagenpeptid zur Anwendung nach einem der vorhergehenden Ansprüche 1 bis 3, wobei das Kollagenpeptid eine Aminosäuresequenz ausgewählt aus der Gruppe bestehend aus SEQ ID No. 1 bis 4, 6 bis 9, 12 bis 14 und 18 bis 20, aufweist.

5. Das Kollagenpeptid zur Anwendung nach Anspruch 1 oder 2, wobei das Kollagenpeptid keine Aminosäure-Modifikation aufweist, insbesondere keine Hydroxylierung aufweist.

6. Das Kollagenpeptid zur Anwendung nach einem der Ansprüche 1 bis 4, wobei das Kollagenpeptid hydroxyliertes Prolin, hydroxyliertes Lysin oder hydroxyliertes Prolin und Lysin aufweist.

7. Das Kollagenpeptid zur Anwendung nach einem der Ansprüche 1 bis 4 oder 6, wobei das Kollagenpeptid an mindestens einem hydroxylierten Lysin glykosyliert ist.

8. Pharmazeutische Zusammensetzung, umfassend ein synthetisches oder rekombinantes Kollagenpeptid mit einem Molekulargewicht in einem Bereich von 0,18 bis 5,0 kDa, welches in der Lage ist, in Bindegewebszellen, insbesondere in Osteoblasten, Chondrocyten und/oder Fibroblasten, die Synthese von extrazellulären Matrixproteinen, insbesondere Kollagen, zu stimulieren und mindestens einen pharmazeutisch akzeptablen Zusatzstoff, wobei das Kollagenpeptid eine Aminosäuresequenz ausgewählt aus der Gruppe bestehend aus SEQ ID No. 1 bis 30 aufweist und wobei keine weiteren Kollagenpeptide in der Zusammensetzung vorliegen.

9. Nahrungsergänzungsmittel, Lebensmittel oder Genussmittel umfassend ein synthetisches oder rekombinantes Kollagenpeptid mit einem Molekulargewicht in einem Bereich von 0,18 bis 5,0 kDa, welches in der Lage ist, in Bindegewebszellen, insbesondere in Osteoblasten, Chondrocyten und/oder Fibroblasten, die Synthese von extrazellulären Matrixproteinen, insbesondere Kollagen, zu stimulieren und mindestens einen Nahrungsmittel-akzeptablen Zusatzstoff, wobei das Kollagenpeptid eine Aminosäuresequenz ausgewählt aus der Gruppe bestehend aus SEQ ID No. 1 bis 30 aufweist und wobei keine weiteren Kollagenpeptide in dem Nahrungsergänzungsmittel, Lebensmittel oder Genussmittel vorliegen.

10. Kosmetisches Produkt, umfassend ein synthetisches oder rekombinantes Kollagenpeptid mit einem Molekulargewicht in einem Bereich von 0,18 bis 5,0 kDa, welches in der Lage ist, in Bindegewebszellen, insbesondere in Osteoblasten, Chondrocyten und/oder Fibroblasten, die Synthese von extrazellulären Matrixproteinen, insbesondere Kollagen, zu stimulieren optional zusammen mit mindestens einem hautverträglichen Zusatzstoff, wobei das Kollagenpeptid eine Aminosäuresequenz ausgewählt aus der Gruppe bestehend aus SEQ ID No. 1 bis 30 aufweist und wobei keine weiteren Kollagenpeptide in dem Produkt vorliegen.

11. Nicht-therapeutisches Verfahren zur optischen Verbesserung der Haut, insbesondere zur Reduktion der Faltenbildung, Verbesserung der Hautelastizität, Erhöhung der Spannkraft der Haut, Erhöhung des Feuchtigkeitsgehalts der Haut, Reduktion von Cellulite und/oder Reduktion von Dehnungsstreifen, insbesondere Schwangerschaftsstreifen, zur Beschleunigung des Wachstums der Nägel und/oder zur Reduktion der Brüchigkeit von Nägeln, zur optischen Verbesserung der Haare, insbesondere zur Verbesserung der Haarqualität, Verminderung von Spliss und/oder Reduktion oder Verzögerung von Haarausfall, wobei dem menschlichen oder tierischen Körper mindestens ein synthetisches oder rekombinantes Kollagenpeptid mit einem Molekulargewicht in einem Bereich von 0,18 bis 5,0 kDa verabreicht wird, welches in der Lage ist, in Bindegewebszellen, insbesondere in Osteoblasten, Chondrocyten und/oder Fibroblasten, die Synthese von extrazellulären Matrixproteinen, insbesondere Kollagen, zu stimulieren und wobei das Kollagenpeptid eine Aminosäuresequenz ausgewählt aus der Gruppe bestehend aus SEQ ID No. 1 bis 30 aufweist.

## Claims

1. A synthetic or recombinant collagen peptide for use in a method for the prevention and/or treatment of bone diseases, cartilage diseases, degenerative joint diseases, diseases of the tendons or ligaments and/or treatment of skin diseases, wherein the collagen peptide has a molecular weight in a region of 0.18 to 5.0 kDa and is able to stimulate the synthesis of extracellular matrix proteins, in particular collagen, in connective tissue cells, in particular in osteoblasts, chondrocytes and/or fibroblasts, and wherein the collagen peptide has an amino acid sequence selected from the group consisting of SEQ ID No. 1 to 30.

2. The collagen peptide for use of claim 1, wherein the collagen peptide has the amino acid sequence SEQ ID No. 1 or 15.

3. The collagen peptide for use of claim 1 or 2, wherein the collagen peptide has the amino acid sequence SEQ ID No. 1, in which at least one proline residue is hydroxylated, in particular wherein the collagen peptide has the amino acid sequence SEQ ID No. 6.

4. The collagen peptide for use of one of the preceding claims 1 to 3, wherein the collagen peptide has an amino acid sequence selected from the group consisting of SEQ ID No. 1 to 4, 6 to 9, 12 to 14 and 18 to 20.

5. The collagen peptide for use of claim 1 or 2, wherein the collagen peptide has no amino acid modification, in particular no hydroxylation.

6. The collagen peptide for use of one of claims 1 to 4, wherein the collagen peptide has hydroxylated proline, hydroxylated lysine or hydroxylated proline and lysine.

7. The collagen peptide for use of one of claims 1 to 4 or 6, wherein the collagen peptide is glycosylated at at least one hydroxylated lysine.

8. A pharmaceutical composition comprising a synthetic or recombinant collagen peptide with a molecular weight in a region of 0.18 to 5.0 kDa, which is able to stimulate the synthesis of extracellular matrix proteins, in particular collagen, in connective tissue cells, in particular in osteoblasts, chondrocytes and/or fibroblasts, and at least one pharmaceutically acceptable additive, wherein the collagen peptide has an amino acid sequence selected from the group consisting of SEQ ID No. 1 to 30 and wherein no further collagen peptides are present in the composition.

9. A food supplement, foodstuff or luxury foodstuff comprising a synthetic or recombinant collagen peptide with a molecular weight in a region of 0.18 to 5.0 kDa, which is able to stimulate the synthesis of extracellular matrix proteins, in particular collagen, in connective tissue cells, in particular in osteoblasts, chondrocytes and/or fibroblasts, and at least one food-acceptable additive, wherein the collagen peptide has an amino acid sequence selected from the group consisting of SEQ ID No. 1 to 30 and wherein no further collagen peptides are present in the food supplement, foodstuff or luxury foodstuff.

10. A cosmetic product comprising a synthetic or recombinant collagen peptide with a molecular weight in a region of 0.18 to 5.0 kDa, which is able to stimulate the synthesis of extracellular matrix proteins, in particular collagen, in connective tissue cells, in particular in osteoblasts, chondrocytes and/or fibroblasts, optionally together with at least one skin-compatible additive, wherein the collagen peptide has an amino acid sequence selected from the group consisting of SEQ ID No. 1 to 30 and wherein no further collagen peptides are present in the product.

11. A non-therapeutic method for optically improving the skin, in particular for reducing the formation of wrinkles, improving skin elasticity, increasing skin tone, increasing the moisture content of the skin, reducing cellulite and/or reducing stretch marks, in particular pregnancy stretch marks, accelerating nail growth and/or reducing nail brittleness, optically improving the hair, in particular improving hair quality, decreasing split ends and/or reducing or delaying hair loss, wherein at least one synthetic or recombinant collagen peptide with a molecular weight in a region from 0.18 to 5.0 kDa is administered to the human or animal body, which is able to stimulate the synthesis of extracellular matrix proteins, in particular collagen, in connective tissue cells, in particular in osteoblasts, chondrocytes and/or fibroblasts, and wherein the collagen peptide has an amino acid sequence selected from the group consisting of SEQ ID No. 1 to 30.

## Revendications

1. Un peptide de collagène synthétique ou recombinant à utiliser dans un procédé de prévention et/ou de traitement de maladies osseuses, de maladies du cartilage, de maladies dégénératives de l'articulation, de maladies des tendons ou des ligaments et/ou de traitement de maladies de la peau, dans lequel le peptide de collagène a un poids moléculaire dans une région de 0,18 à 5,0 kDa et est capable de stimuler la synthèse de protéines de la matrice extracellulaire, en particulier de collagène, dans des cellules de tissu conjonctif, en particulier dans des ostéoblastes, des chondrocytes et/ou des fibroblastes, et dans lequel le peptide de collagène a une séquence d'acides aminés choisie dans le groupe constitué par les séquences SEQ ID No. 1 à 30.

2. Le peptide de collagène à utiliser selon la revendication 1, dans lequel le peptide de collagène a la séquence d'acides aminés SEQ ID No. 1 ou 15.

3. Le peptide de collagène à utiliser selon la revendication 1 ou 2, dans lequel le peptide de collagène a la séquence d'acides aminés SEQ ID No. 1, dans laquelle au moins un résidu de proline est hydroxylé, en particulier dans lequel le peptide de collagène a la séquence d'acides aminés SEQ ID No. 6.

4. Le peptide de collagène à utiliser selon l'une des revendications 1 à 3 précédentes, dans lequel le peptide de collagène a une séquence d'acides aminés choisie dans le groupe constitué par les séquences SEQ ID No. 1 à 4, 6 à 9, 12 à 14 et 18 à 20.

5. Le peptide de collagène à utiliser selon la revendication 1 ou 2, dans lequel le peptide de collagène n'a pas de modification d'acide aminé, en particulier pas d'hydroxylation.

6. Le peptide de collagène à utiliser selon l'une des revendications 1 à 4, dans lequel le peptide de collagène a de la proline hydroxylée, de la lysine hydroxylée ou de la proline et de la lysine hydroxylées.

7. Le peptide de collagène à utiliser selon l'une des revendications 1 à 4 ou 6, dans lequel le peptide de collagène est glycosylé sur d'au moins une lysine hydroxylée.

8. Une composition pharmaceutique comprenant un peptide de collagène synthétique ou recombinant ayant un poids moléculaire dans une région de 0,18 à 5,0 kDa, qui est capable de stimuler la synthèse de protéines de la matrice extracellulaire, en particulier de collagène, dans des cellules de tissu conjonctif, en particulier dans des ostéoblastes, des chondrocytes et/ou des fibroblastes, et au moins un additif pharmaceutiquement acceptable, dans lequel le peptide de collagène a une séquence d'acides aminés choisie dans le groupe constitué par les séquences SEQ ID No. 1 à 30 et dans lequel aucun autre peptide de collagène n'est présent dans la composition.

9. Un complément alimentaire, produit alimentaire ou produit alimentaire de luxe comprenant un peptide de collagène synthétique ou recombinant ayant un poids moléculaire dans une région de 0,18 à 5,0 kDa, qui est capable de stimuler la synthèse de protéines de la matrice extracellulaire, en particulier de collagène, dans des cellules de tissu conjonctif, en particulier dans des ostéoblastes, des chondrocytes et/ou des fibroblastes, et au moins un additif acceptable dans les aliments, dans lequel le peptide de collagène a une séquence d'acides aminés choisie dans le groupe constitué par les séquences SEQ ID No. 1 à 30 et dans lequel aucun autre peptide de collagène n'est présent dans le complément alimentaire, le produit alimentaire ou le produit alimentaire de luxe.

10. Un produit cosmétique comprenant un peptide de collagène synthétique ou recombinant ayant un poids moléculaire dans une région de 0,18 à 5,0 kDa, qui est capable de stimuler la synthèse de protéines de la matrice extracellulaire, en particulier le collagène, dans des cellules de tissu conjonctif, en particulier dans des ostéoblastes, des chondrocytes et/ou des fibroblastes, optionnellement avec au moins un additif compatible avec la peau, dans lequel le peptide de collagène a une séquence d'acides aminés choisie dans le groupe constitué par les séquences SEQ ID No. 1 à 30 et dans lequel aucun autre peptide de collagène n'est présent dans le produit.

11. Un procédé non thérapeutique pour améliorer optique l'aspect de la peau, en particulier pour réduire la formation de rides, améliorer l'élasticité de la peau, augmenter le tonus de la peau, augmenter la teneur en humidité de la peau, réduire la cellulite et/ou réduire les vergetures, en particulier les vergetures de grossesse, accélérer la croissance des ongles et/ou réduire la fragilité des ongles, améliorer optique des cheveux, en particulier améliorer de la qualité des cheveux, diminuer des pointes fourchues et/ou réduire ou retarder de la chute des cheveux, dans lequel au moins un peptide de collagène synthétique ou recombinant ayant un poids moléculaire dans une région de 0,18 et 5,0 kDa est administré au corps humain ou animal, qui est capable de stimuler la synthèse de protéines de la matrice extracellulaire, en particulier de collagène, dans des cellules de tissu conjonctif, en particulier dans des ostéoblastes, des chondrocytes et/ou des fibroblastes, et dans lequel le peptide de collagène a une séquence d'acides aminés choisie dans le groupe constitué par les séquences SEQ ID No. 1 à 30.
